(19) Europäisches Patentamt
European Patent Office
Office européen des brevets

(11) **EP 3 515 530 B1**

(12) **EUROPÄISCHE PATENTSCHRIFT**

(45) Veröffentlichungstag und Bekanntmachung des Hinweises auf die Patenterteilung:
**17.05.2023   Patentblatt 2023/20**

(21) Anmeldenummer: **17772664.3**

(22) Anmeldetag: **19.09.2017**

(51) Internationale Patentklassifikation (IPC):
***A61M 1/36*** *(2006.01)*

(52) Gemeinsame Patentklassifikation (CPC):
**A61M 1/3609; A61M 1/3612; A61M 1/3646; A61M 1/365;** A61M 2230/207

(86) Internationale Anmeldenummer:
**PCT/EP2017/073632**

(87) Internationale Veröffentlichungsnummer:
**WO 2018/054901 (29.03.2018 Gazette 2018/13)**

(54) **STEUERUNGS- UND REGELUNGSEINRICHTUNG SOWIE BEHANDLUNGSVORRICHTUNG ZUM DURCHFÜHREN EINES VERFAHRENS ZUM ENTFERNEN VON BLUT AUS EINEM EXTRAKORPORALEN BLUTKREISLAUF NACH BEENDIGUNG EINER BLUTBEHANDLUNGSSITZUNG**

REGULATING- AND CONTROL-DEVICE AS WELL AS TREATMENT SYSTEM FOR CARRYING OUT A METHOD FOR REMOVING BLOOD FROM AN EXTRACORPOREAL BLOOD CIRCUIT AFTER A BLOOD TREATMENT SESSION HAS ENDED

DISPOSITIF DE COMMANDE ET DE RÉGLAGE AINSI QUE DISPOSITIF DE TRAITEMENT PERMETTANT DE EXÉCUTER UN PROCÉDÉ D'ÉLIMINATION DU SANG RESTANT DANS UN CIRCUIT SANGUIN EXTRACORPOREL À L'ISSUE D'UNE SÉANCE DE TRAITEMENT SANGUIN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.09.2016   DE 102016117725**

(43) Veröffentlichungstag der Anmeldung:
**31.07.2019   Patentblatt 2019/31**

(73) Patentinhaber: **Fresenius Medical Care Deutschland GmbH**
**61352 Bad Homburg (DE)**

(72) Erfinder: **THYS, Martin**
**97508 Grettstadt (DE)**

(74) Vertreter: **Bobbert & Partner**
**Patentanwälte PartmbB**
**Postfach 1252**
**85422 Erding (DE)**

(56) Entgegenhaltungen:
**EP-A1- 2 583 701          WO-A1-2008/028579**
**WO-A1-2010/121750     DE-A1-102011 108 785**

**Beschreibung**

Die vorliegende Erfindung betrifft ein Verfahren zum Entfernen von Blut und/oder eines Blutgemischs aus einem zur Blutbehandlung eines Patienten verwendeten extrakorporalen Blutkreislauf nach Beendigung einer Blutbehandlungssitzung. Sie betrifft ferner eine Steuerungs- und/oder Regelungseinrichtung. Des Weiteren betrifft die vorliegende Erfindung eine medizinische Behandlungsvorrichtung zum Durchführen des erfindungsgemäßen Verfahrens, ein digitales Speichermedium, ein Computerprogramm-Produkt sowie ein Computerprogramm.

Nach dem Ende einer Blutbehandlung wird üblicherweise das Blut, das sich noch im extrakorporalen Kreislauf befindet, mittels Substituierflüssigkeit (im Folgenden auch kurz als Substituat bezeichnet) aus dem extrakorporalen Blutkreislauf in Richtung des Gefäßsystems des Patienten verdrängt und diesem so reinfundiert. Hierbei findet durch Turbulenzen an der Grenzstelle zwischen Substituat und Blut in Schläuchen und Filterfasern häufig eine starke Vermischung dieser beiden Flüssigkeiten statt. Dem Patienten wird bis zur vollständigen Reinfusion des Bluts somit verfahrensbedingt ungewollt auch eine gewisse Menge Substituat infundiert.

Die Infusion von Substituat ist, da im Regelfall die Entfernung von flüssigem Anteil aus dem Blut des Patienten Therapieziel darstellt, unerwünscht und sogar kontraproduktiv, da die vorausgegangene Behandlung u.a. gerade den Zweck hatte, Wasser aus dem Patientenblut zu entfernen Das Entfernen von Blut und/oder eines Blutgemischs aus einem extrakorporalen Blutkreislauf wird z.B. in WO2008/028579 (A1), DE 10 2011 108785 (A1), WO2010/121750 (A1) und EP 2 583 701 (A1) beschrieben.

Es ist Aufgabe der vorliegenden Erfindung, ein weiteres Verfahren sowie geeignete Vorrichtungen zum Entfernen von Blut oder einem Blutgemisch aus einem extrakorporalen Blutkreislauf anzugeben. Zudem ist es eine Aufgabe der Erfindung, ein weiteres digitales Speichermedium, ein weiteres Computerprogramm-Produkt sowie ein weiteres Computerprogramm bereitzustellen, die jeweils in Verbindung mit einem Computersystem die maschinell durchführbaren Schritte des erfindungsgemäßen Verfahrens bewirken.

Die Erfindung wird durch die Merkmale der unabhängigen Ansprüche definiert. Die im Rahmen dieser Beschreibung offenbarten Verfahren fallen als solche nicht unter den beanspruchten Gegenstand der Ansprüche.

Die erfindungsgemäße Aufgabe wird gelöst mittels der Steuerungs- und/oder Regelungseinrichtung mit den Merkmalen des Anspruchs 1, der medizinischen Behandlungsvorrichtung mit den Merkmalen des Anspruchs 9, des digitalen Speichermediums mit den Merkmalen des Anspruchs 11, des Computerprogramm-Produkts mit den Merkmalen des Anspruchs 12, sowie des Computerprogramms mit den Merkmalen des Anspruchs 13.

Erfindungsgemäß wird somit ein Verfahren zum Entfernen von Blut und/oder eines Blutgemischs aus einem zur Blutbehandlung eines Patienten verwendeten extrakorporalen Blutkreislauf mit einem Blutfilter nach Beendigung einer Blutbehandlungssitzung oder einer Blutbehandlung vorgeschlagen. Dabei weist der Blutfilter eine Blutkammer und eine Dialysatkammer auf, zwischen welchen eine Membran angeordnet ist. Die Blutkammer ist zur Blutbehandlung mit einer zur Blutkammer führenden arteriellen Blutleitung und einer von der Blutkammer wegführenden venösen Blutleitung verbunden. Er ist ferner mit einer zur Dialysatkammer führenden Dialysierflüssigkeitszulaufleitung und einer von der Dialysatkammer wegführenden Dialysatablaufleitung verbunden. Das Verfahren umfasst zumindest die Schritte: Verdrängen des Bluts und/oder des Blutgemischs aus der Blutkammer durch Einbringen von Substituat in die arterielle Blutleitung und Erzeugen einer Druckdifferenz im Blutfilter. Dabei wird zumindest zeitweise ein niedrigerer Druck in der Dialysatkammer erzeugt oder sichergestellt als in der Blutkammer.

Die erfindungsgemäße Steuerungseinrichtung, welche auch als Regelungseinrichtung ausgestaltet sein kann, ist geeignet und vorgesehen und/oder programmiert und/oder ausgelegt und/oder konfiguriert zur Durchführung des erfindungsgemäßen Verfahrens im Zusammenwirken mit einer medizinischen Blutbehandlungsvorrichtung. Sie kann gegebenenfalls weitere Einrichtungen wie beispielsweise Speichereinrichtungen, Zugabeeinrichtungen, (vorzugsweise automatisierte) Signalgebungseinrichtungen usw. umfassen.

Die erfindungsgemäße medizinische Behandlungsvorrichtung (im Folgenden auch kurz: Behandlungsvorrichtung) weist optional wenigstens einen extrakorporalen Blutkreislauf mit einem Leitungsinneren auf. Sie ist versehen mit wenigstens einer an oder im extrakorporalen Blutkreislauf angeordneten oder anordenbaren Blutpumpe zum Fördern von Blut innerhalb des Leitungsinneren des extrakorporalen Blutkreislaufs. Zudem weist sie eine erfindungsgemäße Steuerungs- oder Regelungseinrichtung auf.

Ein erfindungsgemäßes, insbesondere digitales, insbesondere nicht-flüchtiges, Speichermedium (hier auch als Träger bezeichnet), insbesondere in Form von Diskette, RAM, ROM, CD, Festplatte, DVD, USB-Stick, Flashcard, SD-Karte oder EPROM, insbesondere mit elektronisch oder optisch auslesbaren Steuersignalen, kann derart mit einem programmierbaren Computer oder Computersystem zusammenwirken, dass die maschinellen Schritte eines hierin beschriebenen erfindungsgemäßen Verfahrens veranlasst werden.

Dabei können alle, einige oder manche der maschinell durchgeführten Schritte des erfindungsgemäßen Verfahrens veranlasst werden.

Ein erfindungsgemäßes Computerprogramm-Produkt weist einen volatilen, flüchtigen oder auf einem maschinenlesbaren Träger gespeicherten Programmcode oder Maschinensteueranweisungen zur Veranlassung der maschinellen Schritte des erfindungsgemäßen Verfahrens, wenn das Computerprogramm-Produkt auf einem Rechner abläuft, auf.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte, ein EPROM und dergleichen sein.

Ein erfindungsgemäßes Computerprogramm weist einen Programmcode auf zur Veranlassung der maschinellen Schritte des erfindungsgemäßen Verfahrens, wenn das Computerprogramm auf einem Computer abläuft.

Dabei können alle, einige oder manche der maschinell durchgeführten Schritte des erfindungsgemäßen Verfahrens veranlasst werden.

Unter einem Computerprogramm-Produkt kann erfindungsgemäß beispielsweise ein auf einem Träger gespeichertes Computerprogramm, eine Signalwelle, ein Embedded System als umfassendes System mit einem Computerprogramm (z. B. ein elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Unter einem Computerprogramm kann erfindungsgemäß beispielsweise ein physikalisches, vertriebsfähiges Software-Produkt verstanden werden, welches ein Programm aufweist.

Auch für das erfindungsgemäße Computerprogramm-Produkt und das erfindungsgemäße Computerprogramm gilt, dass alle, einige oder manche der maschinell durchgeführten Schritte des erfindungsgemäßen Verfahrens veranlasst werden können.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Ein Blutgemisch beschreibt ein Gemisch aus Blut und wenigstens einem weiteren Fluid, insbesondere einer weiteren Flüssigkeit. Ein Blutgemisch ist beispielsweise ein Gemisch aus Blut und Substituat, z.B. einer Lösung, wie z.B. Dialysierflüssigkeit, physiologische Kochsalzlösung etc.

Ein "Substituat" kann beispielsweise jedes zur Verwendung bei einer Blutbehandlung, wie z. B. einer Hämodiafiltration, allgemein bekannte Substituat oder Dialysierflüssigkeit sein, vorzugsweise eine bereits während der Blutbehandlungssitzung verwendete und somit über eine Fluidverbindung bereits in den extrakorporalen Blutkreislauf eingebrachte oder einbringbare Lösung, z.B. isotone Kochsalzlösung, z. B. eine 0,9%ige NaCl-Lösung. Der Begriff "Substituat" kann hierin auch "Austauschflüssigkeit" bedeuten.

Q beschreibt eine Flussrate, hierin kurz auch Fluss genannt. $Q_{UF}$ beschreibt dabei die Flussrate durch eine Ultrafiltrationspumpe (hierin auch kurz: UF-Pumpe), $Q_{BP}$ beschreibt die Flussrate durch eine Blutpumpe, Qsubstituatpumpe beschreibt die Flussrate durch eine Substituatpumpe, $Q_{Fördereinrichtung}$ beschreibt die Flussrate durch eine Fördereinrichtung und $Q_{patient}$ die Flussrate, mit der dem Patienten Blut oder Blutgemisch reinfundiert wird.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale in jeder beliebigen Kombination aufweisen, sofern eine solche für den Fachmann nicht als technisch unmöglich zu erkennen ist.

In einigen beispielhaften Ausführungsformen umfasst die erfindungsgemäße medizinische Behandlungsvorrichtung eine Einrichtung, etwa eine Fördereinrichtung wie z.B. eine Substituatpumpe, Blutpumpe oder Dialysierflüssigkeitspumpe, welche vorgesehen ist zum Einbringen von Substituat in den extrakorporalen Blutkreislauf, z. B. in die arterielle Blutleitung. Die Fördereinrichtung, welche vorgesehen ist zum Einbringen von Substituat, wird im Folgenden auch als Fördereinrichtung für Substituat bezeichnet.

In einigen, beispielhaften erfindungsgemäßen Ausführungsformen werden für das erfindungsgemäße Verfahren ein oder mehrere der folgenden Aktoren oder Fördereinrichtungen verwendet:

- eine Fördereinrichtung für Substituat, insbesondere eine Substituatpumpe und/oder eine Blutpumpe
- ein Filter mit einer semipermeablen Membran und einem dialysierflüssigkeitsseitigen oder geräteseitigen Anschluss
- eine Pumpe, die einen Druck oder Unterdruck aufbaut, welcher einen Transmembranfluss von der Blutseite des Filters zur Dialysatseite des Filters treibt. Diese Pumpe ist in einigen Ausführungsformen identisch mit der Pumpe, die Substituat fördert. In anderen Ausführungsformen sind die beiden genannten Pumpen nicht identisch miteinander, sondern getrennt ausgeführt. In einigen Ausführungsformen ist die Pumpe, die den Druck oder Unterdruck aufbaut eine Ultrafiltrationspumpe (kurz: UF-Pumpe). Die UF-Pumpe erzeugt dabei vorzugsweise einen Unterdruck in der Dialysatkammer.

∘ ein Flusswiderstand. Der Flusswiderstand befindet sich vorzugsweise im extrakorporalen Blutkreislauf auf der Abflussseite der Blutkammer des Blutfilters, so dass durch den Flusswiderstand in Verbindung mit der zuvor genannten Pumpe, die einen Druck aufbaut, ein Überdruck in der Blutkammer entsteht.

Die UF-Pumpe und der Flusswiderstand führen bei geeigneter Ausgestaltung oder Anwendung in der Anwendung des Verfahrens zu einer Druckdifferenz zwischen der Blutseite und der Dialysatseite des Filters, wodurch ein Transmembranfluss von der Blutseite zur Dialysatseite des Filters entsteht. Dabei werden vorzugsweise Wasser und gegebenenfalls niedermolekulare Bestandteile von der Blutseite zur Dialysatseite abgepresst oder -gesaugt während zelluläre Bestandteile auf der Blutseite verbleiben, so dass sich der Hämatokrit auf der Blutseite erfindungsgemäß erhöhen kann.

Wie aus dem Stand der Technik bekannt ist, beginnt die Blutrückgabe in einigen erfindungsgemäßen Ausführungsformen - vorzugsweise sofort - nach Ende der eigentlichen Therapie. Das Patientenblut ist dann vorzugsweise nach ärztlicher Verschreibung (welche eine Zeitangabe, eine qualitative oder quantitative Angabe sein kann) gereinigt und die Wassermenge (bzw. Plasma) im Patientenblut ist entsprechend, z. B. nach Verschreibung, verringert. Die Dauer der Therapie beträgt dabei typischerweise 3 bis 5 Stunden. Die Dauer kann vorab festgelegt sein. Der Ablauf dieser Dauer kann das Ende der Blutbehandlung bedeuten.

Wenn die Blutrückgabe endet, so signalisiert das Gerät dem Bediener dies in einigen Ausführungsformen. Dies kann z. B. dann der Fall sein, wenn der Hämatokrit im extrakorporalen Blutkreislauf (z.B. an einer Erfassungseinrichtung, insbesondere an einem venösen Substituat-Blut-Detektor) bis auf einen vorbestimmten Wert abgesunken ist (z.B. 2%).

In einigen Ausführungsformen wird dem Benutzer das Ende der Blutbehandlung angezeigt oder mitgeteilt. Das erfindungsgemäße Verfahren schließt sich hieran an.

Die Mitteilung des Endes der Blutbehandlung kann den Benutzer z. B. durch Anzeige (z. B. per Monitor) und/oder per Alarm mitgeteilt werden.

Das Ende der Blutbehandlung kann erreicht und/oder angezeigt oder mitgeteilt werden, wenn z. B. das vorab verschriebene oder eingestellte Ultrafiltrationsvolumen entzogen ist.

Das Ende der Blutbehandlung kann erreicht und/oder angezeigt oder mitgeteilt werden, wenn das Behandlungsende mittels User Interface dem Benutzer mitgeteilt wird.

Das Ende der Blutbehandlung kann erreicht und/oder angezeigt oder mitgeteilt werden, wenn Alarmsysteme oder Alarmgrenzen, die während der Blutbehandlung aktiv sind, inaktiviert wurden.

In einigen Ausführungsformen wird während der Ausführung des erfindungsgemäßen Verfahrens dem Patienten kein Blut entnommen. Die Blutbehandlung ist deshalb vor dem Beginn des erfindungsgemäßen Verfahrens vorzugsweise beendet, und es wird kein Blut mehr in den extrakorporalen Blutschlauchsatz eingeführt. Es wird vorzugsweise lediglich bereits vor Beginn des erfindungsgemäßen Verfahrens behandeltes Blut dem Patienten reinfundiert.

Das erfindungsgemäße Verfahren beginnt vorzugsweise nach dem Ende der Blutbehandlung, vorzugsweise mittelbar oder unmittelbar nach deren Ende.

In einigen Ausführungsformen läuft während der Ausführung des erfindungsgemäßen Verfahrens die Blutpumpe rückwärts.

"Rückwärts" kann bedeuten, dass die Blutpumpe in einer Richtung hin zum arteriellen Patientenanschluss oder hin zur arteriellen Patientenschlauchklemme fördert.

"Rückwärts" kann bedeuten, dass die Blutpumpe in einer Richtung fördert, welche der Förderrichtung der Blutpumpe während der Blutbehandlung entgegengesetzt ist.

In einigen Ausführungsformen des erfindungsgemäßen Verfahrens fördert die Blutpumpe eine Spülflüssigkeit, die zum Zweck der Blutreinfusion mit dem extrakorporalen Blutkreislauf verbunden wurde.

In einigen Ausführungsformen des erfindungsgemäßen Verfahrens fördert die Blutpumpe eine Kochsalzlösung, die aus einem Beutel oder anderen Behältnis heraus in den extrakorporalen Blutkreislauf hinein gespeist wird.

In einigen Ausführungsformen des erfindungsgemäßen Verfahrens sind die arterielle Patientenleitung und die venöse Patientenleitung miteinander verbunden.

In einigen Ausführungsformen des erfindungsgemäßen Verfahrens sind die arterielle Patientenleitung und/oder die venöse Patientenleitung mit jeweils einem venösen bzw. einem arteriellen Abschnitt des extrakorporalen Blutkreislaufs oder einer Blutkassette verbunden.

In mehreren beispielhaften Ausführungsformen des erfindungsgemäßen Verfahrens wird die Druckdifferenz zwischen der Blutkammer und der Dialysatkammer des Blutfilters zumindest teilweise durch mindestens eine Pumpe, insbesondere eine Fördereinrichtung für Substituat, eine Ultrafiltrationspumpe, eine Substituatpumpe und/oder eine Blutpumpe erzeugt.

In einigen Ausführungsformen wird in dem erfindungsgemäßen Verfahren die Ultrafiltrationspumpe zumindest zeitweise gleichzeitig mit der Fördereinrichtung für Substituat, der Substituatpumpe und/oder der Blutpumpe betrieben. Auf diese Weise kann in einigen Ausführungsformen eine Druckdifferenz zwischen der Blutkammer und der Dialysatkammer des Blutfilters erzeugt werden.

In bestimmten Ausführungsformen fördert die Fördereinrichtung für Substituat, insbesondere die Blutpumpe und/oder die Substituatpumpe, mit einer Flussrate $Q_{Fördereinrichtung}$, $Q_{BP}$ bzw. $Q_{Substituatpumpe}$ von 20 bis 300 ml/min, vorzugsweise mit 30 bis 280, mit 70 bis 240, und besonders vorzugsweise mit 150 bis 210 ml/min.

In einigen Ausführungsformen fördert die Ultrafiltrationspumpe vorzugsweise mit einem Bruchteil der Flussrate der Fördereinrichtung für Substituat, insbesondere der Blutpumpe und/oder der Substituatpumpe, so dass der Quotient $Q_{UF}/Q_{Fördereinrichtung}$, $Q_{UF}/Q_{BP}$ bzw. QUP/Qsubstituatpumpe vorzugsweise in einem Wertebereich von 0,005 bis 0,9 oder von 0,01 bis 0,8, besonders vorzugsweise von 0,1 bis 0,7 und ganz besonders vorzugsweise von 0,2 bis 0,6 liegt. Dabei liegt $Q_{UF}$ vorzugsweise zwischen 1 und 80 ml/min.

In einigen Ausführungsformen des erfindungsgemäßen Verfahrens fördert die Ultrafiltrationspumpe vorzugsweise zwischen 1 ml/min und 150 ml/min, besonders bevorzugt zwischen 15 ml/min und 150 ml/min.

In einigen Ausführungsformen des erfindungsgemäßen Verfahrens fördert die Ultrafiltrationspumpe vorzugsweise zwischen 15 ml/min und 150 ml/min, besonders bevorzugt zwischen 20 ml/min und 150 ml/min.

In einigen Ausführungsformen des erfindungsgemäßen Verfahrens fördert die Ultrafiltrationspumpe vorzugsweise oberhalb 15 ml/min, besonders bevorzugt oberhalb von 20 ml/min. Dies kann vorteilhaft zur Beschleunigung der Reinfusion beitragen. Zudem kann die Vermischung von Substituat und Blut so vorteilhaft auf eine verglichen mit den Stand der Technik kurze oder kürzere Schlauchstrecke begrenzt werden.

In einigen Ausführungsformen des erfindungsgemäßen Verfahrens werden die hierin angegebenen Förderraten zumindest zeitweise erreicht.

In einigen Ausführungsformen wird die Flussrate oder der Quotient $Q_{Fördereinrichtung}$, $Q_{UF}$ und/oder $Q_{BP}$ automatisch vom Gerät gesteuert oder geregelt.

In einigen Ausführungsformen wird bei Start der Rückgabe $Q_{UF}/Q_{Fördereinrichtung}$, $Q_{ÜF}/Q_{BP}$ bzw. $Q_{UP}/Q_{Substituatpumpe}$ niedrig oder niedriger als zu einem späteren Zeitpunkt eingestellt, da der Hämatokrit der Flüssigkeit im Filter in der Regel noch hoch ist und ein vergleichsweise starker Viskositätsanstieg bzw. Hämatokrit vermieden werden soll. Im weiteren Verlauf wird der vorstehende Quotient vorzugsweise angehoben, da der Hämatokrit der Flüssigkeit im Filter typischerweise zwischenzeitlich abgesunken ist. Der Hämatokrit des Blut-Substituat-Gemisches wird vorzugsweise durch das erfindungsgemäße Verfahren angehoben, damit dem Patienten vorteilhaft möglichst wenig Substituat infundiert wird.

In einigen Ausführungsformen steigt $Q_{UF}/Q_{Fördereinrichtung}$, $Q_{UF}/Q_{BP}$ und/oder $Q_{UP}/Q_{Substituatpumpe}$ mit zunehmendem infundiertem Volumen an Substituat an, vorzugsweise erfolgt der Anstieg monoton, besonders vorzugsweise streng monoton. Die folgende Tabelle gibt für eine Ausführungsform den beispielhaften Zusammenhang zwischen $Q_{UF}/Q_{BP}$ für ein Volumen des extrakorporalen Kreislaufs von etwa 200 ml an.

| Eingebrachtes Substituatvolumen $V_{substituat}$ [ml], kumulativ | $Q_{UF}/Q_{BP}$ |
|---|---|
| 0 | z.B. 0,1; typisch: 0,01 bis 0,3 |
| 40 | z.B. 0,1; typisch: 0,01 bis 0,3 |
| 80 | z.B. 0,3; typisch: 0,1 bis 0,5 |
| 120 | z.B. 0,5; typisch: 0,1 bis 0,7 |
| 160 | z.B. 0,6; typisch: 0,3 bis 0,8 |
| 200 | z.B. 0,7; typisch: 0,5 bis 0,8 |
| 240 | z.B. 0,7; typisch: 0,5 bis 0,8 |
| 280 | z.B. 0,7; typisch: 0,5 bis 0,8 |

In einer Ausführungsform des erfindungsgemäßen Verfahrens ist der gewünschte Fluss des Substituats durch den Bediener einstellbar. Der Fluss zum Patienten ($Q_{Patient}$) stellt sich vorzugsweise nach folgender Regel ein: $Q_{Patient}$ = $Q_{Fördereinrichtung}$ - $Q_{UF}$, insbesondere $Q_{Patient}$ = $Q_{BP}$ - $Q_{UF}$, wobei die Blutpumpe hier exemplarisch als alleinige Förderpumpe für das Substituat angenommen wurde.

In einigen Ausführungsformen des erfindungsgemäßen Verfahrens ist der gewünschte Fluss in den Patienten ($Q_{PatientDesired}$) durch den Bediener einstellbar und Gerät errechnet, z. B. wiederkehrend oder stetig, $Q_{Fördereinrichtung}$ bzw. $Q_{BP}$ nach der Regel:

$$Q_{\text{Fördereinrichtung}} = Q_{\text{PatientDesired}} + Q_{\text{UF}},$$

$$\text{insbesondere } Q_{\text{BP}} = Q_{\text{PatientDesired}} + Q_{\text{UF}}.$$

In einigen Ausführungsformen des erfindungsgemäßen Verfahrens wird $Q_{\text{UF}}/Q_{\text{Fördereinrichtung}}$, $Q_{\text{UF}}/Q_{\text{BP}}$ und/oder $Q_{\text{UF}}/Q_{\text{Substituatpumpe}}$ in Abhängigkeit von der Menge der bisher geförderten Menge an Substituat gesteuert (z.B. im Sinne einer steigenden Rampe).

In einigen Ausführungsformen des erfindungsgemäßen Verfahrens wird $Q_{\text{UF}}/Q_{\text{Fördereinrichtung}}$, $Q_{\text{UF}}/Q_{\text{BP}}$ und/oder $Q_{\text{UF}}/Q_{\text{Substituatpumpe}}$ in Abhängigkeit von der Menge der bisher geförderten Menge an Substituat und dem (z.B. vom Füllen des Systems) bekannten Volumen des extrakorporalen Blutkreislaufs gesteuert (steigende Rampe). Beispielsweise nimmt $Q_{\text{UF}}/Q_{\text{Fördereinrichtung}}$ des geförderten Substituats monoton zu, vorzugsweise streng monoton zu, besonders vorzugsweise ist $Q_{\text{UF}}/Q_{\text{Fördereinrichtung}}$ proportional zum bereits geförderten Substituatvolumen.

In einigen Ausführungsformen des erfindungsgemäßen Verfahrens wird $Q_{\text{UF}}/Q_{\text{Fördereinrichtung}}$, $Q_{\text{UF}}/Q_{\text{BP}}$ und/oder $Q_{\text{UF}}/Q_{\text{Substituatpumpe}}$ in Abhängigkeit von einer Verschreibung oder akuten Bedürfnissen des Patienten individuell gesteuert.

In einigen Ausführungsformen des erfindungsgemäßen Verfahrens wird $Q_{\text{UF}}/Q_{\text{Fördereinrichtung}}$, $Q_{\text{UF}}/Q_{\text{BP}}$ und/oder $Q_{\text{UF}}/Q_{\text{Substituatpumpe}}$ während der Ausführung des Verfahrens variiert, beispielsweise um den Hämatokrit z. B. in der venösen Blutleitung auf einen vorbestimmten Wert zu regeln.

In einigen Ausführungsformen des erfindungsgemäßen Verfahrens wird $Q_{\text{UF}}/Q_{\text{Fördereinrichtung}}$, $Q_{\text{UF}}/Q_{\text{BP}}$ und/oder $Q_{\text{UF}}/Q_{\text{Substituatpumpe}}$ variiert, um den Hämatokrit im extrakorporalen Blutkreislauf auf einen Wunschwert (typischerweise zwischen 30 und 60%) zu regeln. Als Eingangsgröße des Reglers kann beispielsweise der blutseitige Fließdruck des Filters herangezogen werden (z.B. $p_{\text{Filter\_längs}}$ = PräFilter (PostPump) Druck - PostFilter(venös) Druck, also der Druck, der zwischen Pumpe und Filtereingang herrscht, abzüglich des Drucks, der in der venösen Leitung stromab des Filters herrscht). Der Flusswiderstand ist dabei proportional zur Viskosität der Flüssigkeit im extrakorporalen Blutkreislauf. Die Viskosität ist proportional zum Hämatokrit des Blutes im Filter.

In einigen Ausführungsformen des erfindungsgemäßen Verfahrens wird $Q_{\text{UF}}/Q_{\text{Fördereinrichtung}}$, $Q_{\text{UF}}/Q_{\text{BP}}$ und/oder $Q_{\text{UF}}/Q_{\text{Substituatpumpe}}$ in Abhängigkeit des mittels der Erfassungseinrichtung, insbesondere mittels des venösen Substituat-Blut-Detektors, bestimmten Hämatokrits, etwa in der venösen Blutleitung, und in Abhängigkeit eines vorbestimmten einzusparenden Substituatvolumens geregelt. Beispielsweise gibt der Bediener eine gegenüber dem Stand der Technik einzusparende Menge an Substituat vor und $Q_{\text{UF}}/Q_{\text{Fördereinrichtung}}$, $Q_{\text{UF}}/Q_{\text{BP}}$ und/oder $Q_{\text{UF}}/Q_{\text{Substituatpumpe}}$ wird in Abhängigkeit des Hämatokrits der Flüssigkeit, etwa in der venösen Patientenleitung (z.B. detektiert durch einen optischen Detektor, z.B. einen venösen Substituat-Blut-Detektor), variiert oder geregelt.

In einigen Ausführungsformen des erfindungsgemäßen Verfahrens wird bei simultaner arterieller und venöser Reinfusion $Q_{\text{UF}}/Q_{\text{Fördereinrichtung}}$, $Q_{\text{UP}}/Q_{\text{BP}}$ und/oder $Q_{\text{UF}}/Q_{\text{Substituatpumpe}}$ in Abhängigkeit des Hämatokrits der Flüssigkeit in der arteriellen Patientenleitung derart eingestellt, dass der Hämatokrit der Flüssigkeit, welche durch die venöse Patientenleitung in den Patienten fließt, vergleichsweise sehr hoch ist (z.B. >50%, bevorzugt >55%, besonders bevorzugt etwa 60%). Beispielsweise kann die Flüssigkeit aus der arteriellen Patientenleitung mit einem vergleichsweise niedrigen Hämatokrit (z.B. 10%) sich dann bei der Reinfusion im Patientengefäß mit der Flüssigkeit aus der venösen Patientenleitung, die einen hohen Hämatokrit (z.B. 60%) aufweist, vermischen, so dass sich im Patientengefäß ein Hämatokrit mit einem Wert (z.B. <60%) ergibt, der zwischen den beiden Werten für den Hämatokrit in der venösen bzw. der arteriellen Patientenleitung liegt. Auf diese Weise kann in einigen Ausführungsformen vorteilhaft eine besonders geringe Reinfusion von Substrat erreicht werden.

In einigen Ausführungsformen des erfindungsgemäßen Verfahrens wird $Q_{\text{UF}}/Q_{\text{Fördereinrichtung}}$, $Q_{\text{UP}}/Q_{\text{BP}}$ und/oder $Q_{\text{UP}}/Q_{\text{Substituatpumpe}}$ derart eingestellt, dass in Summe mit dem bereits vor der Blutrückgabe (während der eigentlichen Behandlung) dem Blut entzogenen Wassermenge sich der Wert aus der Verschreibung ergibt

In einigen Ausführungsformen kann das erfindungsgemäße Verfahren vorteilhafterweise bei jedem bekannten Reinfusionsverfahren angewendet werden (z.B. NaCl, Online, Online simultan etc.).

In manchen erfindungsgemäßen Ausführungsformen dient das Verfahren dem teilweisen, in anderen dem vollständigen Entfernen von Blut aus einem zur Blutbehandlung eines Patienten verwendeten Blutfilter und/oder Blutkreislauf nach Beendigung der Blutbehandlungssitzung.

Der verwendete Blutfilter ist in einigen erfindungsgemäßen Ausführungsformen ein Hämodialysator oder ein Hämofilter.

Die zwischen Blutkammer und Dialysatkammer angeordnete Membran ist in bestimmten erfindungsgemäßen Ausführungsformen eine semipermeable Membran.

In gewissen beispielhaften erfindungsgemäßen Ausführungsformen führt die venöse Blutleitung von der Blutkammer des Blutfilters zu einer venösen Blutkammer (hierin auch als venöse Luftabscheidekammer bezeichnet) und/oder einer venösen Konnektionsstelle oder -einrichtung.

In bestimmten beispielhaften erfindungsgemäßen Ausführungsformen führt die arterielle Blutleitung von einer arteriellen Konnektionsstelle oder -einrichtung bis zur Blutkammer des Blutfilters.

In manchen beispielhaften erfindungsgemäßen Ausführungsformen ist die Steuerungs- und/oder Regelungseinrichtung konfiguriert, um im Zusammenwirken mit einer medizinischen Blutbehandlungsvorrichtung eine nicht-erfindungsgemäße Blutbehandlung und eine sich anschließende erfindungsgemäße Verdrängung des Bluts aus der Blutkammer mittels eingebrachten Substituats zu bewirken.

In gewissen beispielhaften erfindungsgemäßen Ausführungsformen sind die medizinische Behandlungsvorrichtung und/oder der Blutfilter mit einer Blutkassette verbunden.

In einigen beispielhaften erfindungsgemäßen Ausführungsformen des Verfahrens umfasst dieses das Erfassen einer qualitativen Änderung des Inhalts des Leitungsinneren des extrakorporalen Blutkreislaufs.

In manchen erfindungsgemäßen Ausführungsformen des Verfahrens wird durch Betreiben einer Fördereinrichtung, etwa der Blutpumpe, der Substituatpumpe oder der Dialysierflüssigkeitspumpe, eine vorbestimmte Menge an Substituat in das Leitungsinnere des extrakorporalen Blutkreislaufs eingebracht.

In einigen erfindungsgemäßen Ausführungsformen des Verfahrens wird Substituat (alternativ als Substituatflüssigkeit zu bezeichnen) gefördert, bis die Erfassungseinrichtung in vorbestimmtem Maße Substituat im Leitungsinneren des extrakorporalen Blutkreislaufs erfasst.

In manchen erfindungsgemäßen Ausführungsformen des Verfahrens ist die Erfassungsvorrichtung mit vorgegebener Distanz zu einer venösen Zugangseinrichtung angeordnet. Das Verfahren umfasst in diesen Ausführungsformen ferner das Fördern des Inhalts des Leitungsinneren des extrakorporalen Blutkreislaufs über die vorgegebene Distanz bis zur venösen Zugangseinrichtung, nachdem Dialysat an der Erfassungseinrichtung erkannt wurde.

In bestimmten erfindungsgemäßen Ausführungsformen des Verfahrens wird im Leitungsinneren des extrakorporalen Blutkreislaufs enthaltenes Blut über die venöse Zugangseinrichtung in das Gefäßsystem des Patienten eingebracht.

Das erfindungsgemäße Verfahren umfasst in einigen erfindungsgemäßen Ausführungsformen ferner das Eintragen oder Einbringen von Luft in den extrakorporalen Blutkreislauf, beispielsweise nach Beenden einer Blutbehandlungssitzung und/oder nach dem teilweisen oder vollständigen Entfernen von Blut aus dem extrakorporalen Blutkreislauf.

Eine "Blutbehandlungssitzung" kann beispielsweise eine Behandlungseinheit mittels Hämodialyse, Hämofiltration, Hämodiafiltration und/oder eines Zellseparationsverfahrens und auf die Behandlung und/oder Reinigung von Blut gerichtet sein. Zum Durchführen einer solchen Blutbehandlung wird eine geeignete Blutbehandlungsvorrichtung verwendet.

Eine zum Durchführen des erfindungsgemäßen Verfahrens geeignete medizinische Behandlungsvorrichtung weist in manchen erfindungsgemäßen Ausführungsformen optional einen extrakorporalen Blutkreislauf mit einem Leitungsinneren, wenigstens eine Fördereinrichtung zum Einbringen und/oder Fördern wenigstens zweier Fluide im Leitungsinneren des extrakorporalen Blutkreislaufs, und beispielsweise eine Einrichtung zum Behandeln des Bluts des Patienten, wie einen oder mehrere Blutfilter und/oder einen oder mehrere Dialysatoren und/oder einen oder mehrere Adsorber, auf oder ist hiermit verbunden. Sie kann ferner Behälter zum Aufbewahren von Fluiden, Elemente zum Einbringen der Fluide, wie beispielsweise Schlauchelemente und/oder Ventile, sowie weitere Einrichtungen, wie z. B. eine Luftabscheidekammer zum Entfernen von Luft aus dem Blut während der Blutbehandlung und/oder Sensoren und/oder Detektoren zum Erfassen verschiedener relevanter Parameter, wie beispielsweise einen Druck im extrakorporalen Blutkreislauf, aufweisen.

Fördereinrichtungen, wie hierin genannt, schließen Membranpumpen, Schlauchpumpen, Rollenpumpen usw. ein. Die Blutpumpe, eine Substituatpumpe und/oder eine Dialysierflüssigkeitspumpe können z. B. als Schlauchpumpe oder Rollenpumpe ausgeführt sein. Es kann aber jeweils wiederum auch ein anderer Pumpentyp, z. B. eine Membranpumpe, eingesetzt werden.

Bei einer Fördereinrichtung für Dialysierflüssigkeit oder Substituat kann es sich um eine "zweite" Fördereinrichtung handeln, also eine Fördereinrichtung, welche nicht mit der Blutpumpe identisch ist. Die Blutpumpe kann jedoch auch derart ausgestaltet sein, dass sie sowohl die für eine Blutpumpe typische Funktion ausführt als auch die Funktion des Einbringens von Substituat in das Leitungsinnere und/oder das Fördern von Leitungsinhalt erfüllt. Wenn hierin allein der besseren Lesbarkeit wegen von einer Fördereinrichtung für Substituat die Rede ist, so ist darunter die Blutpumpe oder eine sich hiervon unterscheidende Fördereinrichtung zu verstehen. Beide Varianten sind gleichermaßen von der vorliegenden Erfindung umfasst.

Das erfindungsgemäße Verfahren umfasst in gewissen erfindungsgemäßen Ausführungsformen den Schritt des Einbringens oder Eintragens von Luft in das Leitungsinnere des extrakorporalen Blutkreislaufs, um diesen von Flüssigkeit zu entleeren, z.B. durch Betreiben der Blutpumpe. Die Luft kann beispielsweise Atmosphärenluft sein. Die vorliegende Erfindung soll jedoch nicht auf die alleinige Verwendung von Luft beschränkt sein, sondern ferner alle, für die Zwecke der vorliegenden Erfindung geeigneten, gasförmigen Fluide anstelle von Luft einschließen.

Das "Eintragen von Luft in das Leitungsinnere des extrakorporalen Blutkreislaufs" nach Beenden der Blutbehandlungssitzung kann ausschließlich oder unterstützend durch die Blutpumpe, durch die zweite Fördereinrichtung oder durch eine Druckluftquelle erfolgen.

Kombinationen der vorgenannten Optionen sind erfindungsgemäß ebenfalls umfasst, ebenso ein passives Eindringenlassen von Luft.

Das "Einbringen von Substituat in das Leitungsinnere des extrakorporalen Blutkreislaufs" erfolgt, wie vorstehend ausgeführt, in einigen erfindungsgemäßen Ausführungsformen durch Betreiben der Fördereinrichtung für Substituat, insbesondere der Blutpumpe und/oder der Substituatpumpe.

Die Blutpumpe kann Substituat fördern, indem sie selbiges aus einer Zuleitung zu einem Behälter für das Substituat ansaugt, die stromaufwärts der Saugseite der Blutpumpe in den extrakorporalen Blutkreislauf mündet. Hierzu kann beispielsweise eine im arteriellen Zweig des extrakorporalen Blutkreislaufs vorgesehene Mündung mit Schlauchklemme vorgesehen sein.

Ist vorgesehen, dass die Blutpumpe sowohl Blut als auch Substituat in den extrakorporalen Blutkreislauf einbringt und fördert, kann das erfindungsgemäße Verfahren mit nur einer Pumpe durchgeführt werden. Obwohl eine solche weiter bevorzugte Ausführungsform von der vorliegenden Erfindung umfasst ist, werden im Folgenden Ausführungsformen beschrieben, bei denen eine Blutpumpe und eine zweite Fördereinrichtung eingesetzt werden. Die folgende Beschreibung soll ein Verständnis der der vorliegenden Erfindung zugrunde liegenden Prinzipien und Funktionen der einzelnen Komponenten vereinfachen. Vorzugsweise wird das Verfahren mit Hilfe einer UF-Pumpe ausgeführt, die einen Unterdruck in der Dialysatkammer erzeugen kann.

In einer bevorzugten erfindungsgemäßen Ausführungsform ist das Erfassen einer qualitativen Änderung des Inhalts des Leitungsinneren des extrakorporalen Blutkreislaufs mit Hilfe wenigstens einer in oder an einem Abschnitt des extrakorporalen Blutkreislaufs angeordneten Erfassungseinrichtung umfasst.

Die "qualitative Änderung" kann sich auf einen oder mehrere Bereiche oder Abschnitte des extrakorporalen Blutkreislaufs beziehen, beispielsweise einen Bereich oder Abschnitt, in welchem die Erfassungseinrichtung vorliegt.

Eine "qualitative Änderung des Inhalts des Leitungsinneren" schließt eine Änderung der Zusammensetzung des Inhalts des Leitungsinneren, wie beispielsweise eine Änderung der einzelnen Anteile an Blut und/oder Substituat im Leitungsinneren oder einem Abschnitt hiervon, bezogen aufeinander, ein. Auch das Fehlen eines vormals vorhandenen Fluids kann eine Änderung der Zusammensetzung darstellen. Eine qualitative Änderung kann auch ein Übergang von Blut zu Substituat sein. Solche Änderungen können z. B. leicht anhand einer optischen Änderung des Inhalts, wie einer Aufhellung oder einer Verdunkelung des Inhalts oder ein Änderung der Farbe, etwa ein Umschlag nach Rot oder ein Zunahme des Rot-Tons, erfasst werden.

Die in einem Abschnitt des extrakorporalen Blutkreislaufs angeordnete "Erfassungseinrichtung" kann z. B. ein optischer Sensor sein, der eine optische Änderung des Inhalts des Leitungsinneren oder eine Eigenschaft des Inhalts erfasst. Weitere geeignete Sensoren schließen Drucksensoren, Leitfähigkeitssensoren und Sensoren zum Erfassen einer Änderung der Dichte des Inhalts des Leitungsinneren des extrakorporalen Blutkreislaufs ein, ohne darauf beschränkt zu sein. In einigen Ausführungsformen ist eine Erfassungseinrichtung ein Substituat-Blut-Detektor oder umfasst diesen.

Der "Abschnitt des extrakorporalen Blutkreislaufs" kann ein arterieller und/oder venöser Abschnitt des extrakorporalen Blutkreislaufs sein. Der "arterielle Abschnitt" bezeichnet einen Abschnitt des extrakorporalen Blutkreislaufs, durch den das Blut aus dem Gefäßsystem des Patienten in Richtung zur Blutbehandlungseinrichtung oder zum Blutfilter strömt. Der "venöse Abschnitt" bezeichnet den Abschnitt des extrakorporalen Blutkreislaufs, durch den das Blut aus der Blutbehandlungseinrichtung oder aus dem Blutfilter zurück zum Gefäßsystem des Patienten strömt.

In einer ebenfalls bevorzugten Weiterentwicklung des erfindungsgemäßen Verfahrens umfasst der extrakorporale Blutkreislauf wenigstens eine mit einem Abschnitt des Gefäßsystems des Patienten konnektierbare Zugangseinrichtung, und das Verfahren umfasst ein Dekonnektieren des extrakorporalen Blutkreislaufs vom Gefäßsystem des Patienten, insbesondere im Bereich einer ersten, beispielsweise arteriellen, Zugangseinrichtung, insbesondere an einem Ende des extrakorporalen Blutkreislaufs.

Das "Dekonnektieren des extrakorporalen Blutkreislaufs vom Gefäßsystem des Patienten" bedeutet das Unterbrechen einer Verbindung zwischen dem extrakorporalen Blutkreislauf und dem Gefäßsystem des Patienten in einem Abschnitt des extrakorporalen Blutkreislaufs, beispielsweise an einem Ende hiervon. Dabei kann die Unterbrechung sowohl am arteriellen als auch am venösen Abschnitt erfolgen, wobei in der vorliegenden Erfindung ein Dekonnektieren des arteriellen Abschnitts des extrakorporalen Blutkreislaufs bevorzugt ist.

Unter einem Dekonnektieren im "Bereich der ersten Zugangseinrichtung" kann z. B. das Herausziehen der arteriellen Konnektionsnadel eines Double-Needle-Zugangs verstanden werden.

Unter einem Dekonnektieren kann auch das Unterbrechen der Strömungsverbindung zwischen dem arteriellen Abschnitt des extrakorporalen Blutkreislaufs und der arteriellen Konnektionsnadel verstanden werden.

Bei der Single-Needle-Variante kann das Unterbrechen der Verbindung zwischen dem arteriellen Schenkel des "Y"-förmigen Abschnitts des extrakorporalen Blutkreislaufs und der einzigen mit dem Gefäßsystem des Patienten verbundenen Konnektionsnadel verstanden werden. Das offene Lumen des arteriellen Schenkels des Y-Stücks kann nach seinem Abtrennen auf beliebige Weise (manuell, maschinell, automatisch, usw.) verschlossen werden.

Alternativ oder zusätzlich kann Gleiches auch für den venösen Abschnitt des extrakorporalen Blutkreislaufs und den venösen Zugang zum Gefäßsystem des Patienten angewandt werden.

Eine "Zugabestelle des extrakorporalen Blutkreislaufs für Substituat in das Leitungsinnere des extrakorporalen Blutkreislaufs" kann in dem arteriellen und/oder dem venösen Abschnitt des extrakorporalen Blutkreislaufs angeordnet sein. Bevorzugt ist die "Zugabestelle" in einem Abschnitt des extrakorporalen Blutkreislaufs angeordnet, der stromaufwärts der Blutbehandlungseinrichtung, wie beispielsweise des Blutfilters, durchströmt wird.

Geeignete Beispiele für eine Zugabestelle schließen ein Öffnungs-/Verschlussventil, einen Absperrhahn, eine zuschaltbare Nebenleitung eines verzweigten Abschnitts des extrakorporalen Blutkreislaufs usw. ein.

Eine "vorbestimmte Substituatmenge oder Dialysierflüssigkeitsmenge" kann einem bestimmten Fördervolumen und/oder einer bestimmten Förderstrecke des Inhalts entlang des Leitungsinneren des extrakorporalen Blutkreislaufs entsprechen und beispielsweise durch Betreiben einer Membranpumpe erfolgen.

Die Substituatmenge oder Dialysierflüssigkeitsmenge kann vorzugsweise als Größe, zum Beispiel als Volumen mit vorgegebener Maßzahl und Einheit, vorbestimmt sein. Die absolute Größe der Substituatmenge kann vorzugsweise beispielsweise in einer Steuereinrichtung der erfindungsgemäßen Behandlungsvorrichtung hinterlegt und/oder eingebbar sein. Die Substituatmenge kann vorzugsweise im Rahmen der technischen Genauigkeit exakt gefördert werden.

Um eine exakte Menge an Substituat vorzubestimmen, können zum Beispiel technische Spezifikationen des eingesetzten extrakorporalen Blutkreislaufs, wie beispielsweise die Innenvolumina des Schlauchsatzes, in der Steuereinrichtung gespeichert oder in diese eingegeben werden. Anhand der technischen Spezifikationen der einzelnen Komponenten des extrakorporalen Blutkreislaufs können zum Beispiel eine erforderliche Förderzeit und/oder ein Fördervolumen berechnet werden.

Eine "begrenzte Substituatmenge oder Dialysierflüssigkeitsmenge" kann eine, zum Beispiel anhand von Erfahrungswerten des Bedienpersonals ausgewählte, Menge an Substituat oder Dialysierflüssigkeit sein. Vorzugsweise kann eine begrenzte Menge eingebracht und so lange gefördert werden, bis an einer weiteren Erfassungseinrichtung das Auftreten von Substituat im Leitungsinneren des extrakorporalen Blutkreislaufs erfasst wird. Eine begrenzte Substituatmenge muss daher nicht genau bekannt sein und/oder einem bestimmten Fördervolumen entsprechen. Eine begrenzte Substituatmenge kann jedoch indirekt durch das Innenvolumen der von der Substituatmenge durchströmten Komponenten des extrakorporalen Blutkreislaufs, insbesondere das Innenvolumen des Abschnitts von der Zugabestelle für Substituat und/oder der Blutbehandlungseinrichtung bis zu einer weiteren Erfassungseinrichtung begrenzt sein. Das Volumen ist dabei somit bestimmt im Sinne von "begrenzt", ohne jedoch exakt bekannt zu sein, und ohne beispielsweise in Millilitern angegeben werden zu können und/oder ohne in einer Steuerung hinterlegt worden oder eingebbar zu sein. Das Einbringen einer begrenzten Substituatmenge kann zum Beispiel von Vorteil sein, wenn der Filtertyp einer Blutbehandlungseinrichtung oder deren Fassungsvermögen nicht bekannt oder nicht richtig angegeben ist.

Dabei kann das Substituat aus einem dafür vorgesehenen Vorratsbehälter über entsprechende Leitungssysteme des extrakorporalen Blutkreislaufs an der Zugabestelle für das Substituat in den extrakorporalen Blutkreislauf eingebracht werden.

Die "Erfassungseinrichtung" ist wie vorstehend definiert und kann beispielsweise im venösen Abschnitt des extrakorporalen Blutkreislaufs, z. B. zwischen der Blutbehandlungseinrichtung und der venösen Zugangseinrichtung zum Gefäßsystem des Patienten und insbesondere zwischen einer Tropfkammer im venösen Abschnitt und der venösen Zugangseinrichtung, angeordnet sein.

Die Erfassungseinrichtung kann das Auftreten von Substituat in einem bestimmten Abschnitt des Leitungsinneren des extrakorporalen Blutkreislaufs beispielsweise anhand einer optischen Änderung des Inhalts des Leitungsinneren erkennen.

Wenn die Erfassungseinrichtung das Auftreten von Luft oder Substituat in dem Leitungsinneren des extrakorporalen Blutkreislaufs erkennt, kann das Fördern des "Substituat-Blut-Inhalts" gestoppt werden.

Dies kann durch Stoppen der entsprechenden Fördereinrichtung erfolgen.

Ferner ist in einer weiteren Ausführungsform des erfindungsgemäßen Verfahrens bevorzugt, die Erfassungseinrichtung mit vorgegebener Distanz zu einer zweiten Zugangseinrichtung anzuordnen und den Inhalt des Leitungsinneren über die vorgegebene Distanz bis zur Zugangseinrichtung zu fördern, nachdem Substituat oder eine vorbestimmte Transmission oder Lichttransmission, ein vorbestimmter Farbton oder eine vorbestimmte Farbänderung an der Erfassungseinrichtung erkannt wurde.

In einer weiter bevorzugten Ausführungsform des erfindungsgemäßen Verfahrens wird das im Leitungsinneren des extrakorporalen Blutkreislaufs enthaltene Blut - insbesondere im Wesentlichen vollständig - über die zweite Zugangseinrichtung in das Gefäßsystem des Patienten rückgeführt. Der Begriff "im Wesentlichen vollständig rückgeführt" meint hierbei, dass das im Leitungsinneren des extrakorporalen Blutkreislaufs vorhandene Blut nahezu rückstandsfrei aus dem extrakorporalen Blutkreislauf entfernt wird. Die gegebenenfalls im extrakorporalen Blutkreislauf aus technischen Gründen wie Benetzungsverhalten oder in der Tropfkammer verbleibenden Blutrückstände sind dabei als vernachlässigbar gering anzusehen.

Das "Rückführen von Blut in das Gefäßsystem des Patienten" kann erfolgen, wenn ein Ende des extrakorporalen Blutkreislaufs, wie beispielsweise das Ende des venösen Abschnitts, z. B. die venöse Konnektionsnadel, mit dem Gefäßsystem des Patienten konnektiert ist. Diese Verbindung kann nach Beenden der Blutbehandlungssitzung beibehalten oder erneut hergestellt werden.

Da mit der erfindungsgemäßen Behandlungsvorrichtung das vorstehend beschriebene erfindungsgemäße Verfahren durchführbar ist, wird zur Vermeidung von Wiederholungen auf die vorstehend beschriebenen Ausführungen derselben verwiesen.

Eine Weiterentwicklung der erfindungsgemäßen Behandlungsvorrichtung sieht das Anordnen wenigstens einer Erfassungseinrichtung zum Erfassen wenigstens einer Änderung des Inhalts des Leitungsinneren des extrakorporalen Blutkreislaufs oder einer Eigenschaft des Inhalts in einem Abschnitt des extrakorporalen Blutkreislaufs vor. Bei einer Eigenschaft des Inhalts kann es sich um eine Zusammensetzung, eine physikalische, chemische oder biologische Größe, beispielsweise eine Transparenz, einen pH-Wert und vieles dergleichen mehr handeln. Eine derartige Erfassungseinrichtung kann der vorstehend beschriebenen entsprechen, so dass zur Vermeidung von Wiederholungen auf deren obige Beschreibung verwiesen wird.

Eine Behandlungsvorrichtung gemäß der vorliegenden Erfindung kann, ohne darauf beschränkt zu sein, zum Durchführen einer Hämodialyse, einer Hämofiltration, einer Hämodiafiltration und/oder Separationsverfahren geeignet und/oder konfiguriert sein.

Mittels mancher erfindungsgemäßer Ausführungsformen können eine oder mehrere der hierin genannten Vorteile erzielbar sein.

In einigen Ausführungsformen ist das Verfahren gegenüber dem Stand der Technik dahingehend geändert, dass während des Reinfusionsvorgangs über den Filter dem Blut-Substituatgemisch, welches durch die Verdrängung des Blutes durch Substituat entstanden ist, Wasser entzogen wird. Das Blut-Substituatgemisch, welches den Filter in Richtung zum Patienten verlässt, enthält dadurch in einigen Ausführungsformen weniger Substituat.

Für einen Patienten mit Niereninsuffizienz ist der Netto-Wasserentzug in der gesamten Behandlung (inkl. Reinfusion) üblicherweise ein entscheidender Behandlungsparameter. Um über die gesamte Behandlung eine bestimmte Netto-Menge Wasser aus dem Patientenblut zu entfernen wird gewöhnlich das während der Reinfusion dem Patienten zusätzlich zum Blut notwendigerweise ebenfalls infundierte Wasser bereits zuvor in der Behandlung dem Patientenblut entzogen.

Mit steigender Menge des in der eigentlichen Behandlung aus dem Patientenblut entfernten Wassers nimmt häufig die Belastung des Kreislaufs des Patienten erheblich zu. Dieses führt regelmäßig zu physiologischen Problemen während der Behandlung. Die während der Reinfusion infundierte Flüssigkeitsmenge, die wie vorstehend beschrieben bereits vor Beendigung der Behandlung ebenfalls entzogen wurde, trägt ebenfalls regelmäßig zu physiologischen Problemen bei. In einigen erfindungsgemäßen Ausführungsformen können derartige physiologische Probleme vorteilhaft vermieden oder verringert werden.

Das erfindungsgemäße Verfahren kann in einigen Ausführungsformen einen, mehrere oder alle der oben oder im Folgenden genannten Vorteile mit sich bringen:

- Verminderter Anteil von Substituat im Blut-Substituatgemisch, welches dem Patienten während der Reinfusion infundiert wird. Dabei kann die während der dem erfindungsgemäßen Verfahren vorangegangenen Behandlung zu entfernende Wassermenge verringert werden.
- Verbesserte Kreislaufstabilität des Patienten (insbesondere bei pädiatrischen Patienten)
- Verminderte Menge des vaskulären Gesamtflüssigkeitseintrags während der Reinfusion
- Verringerte Dauer des Reinfusionsvorgangs
- Kosteneinsparung
- Verringerter Blutverlust des Patienten durch weniger Restblut, das im Stand der Technik nach der Reinfusion im extrakorporalen Blutkreislauf verbleibt. Im Stand der Technik ergibt sich häufig eine Abwägung, ob die Blutrückgabe möglichst vollständig erfolgen soll, wodurch viel Substituat infundiert wird, oder ob möglichst wenig Substituat infundiert werden soll, was mit einer weniger effizienten Blutrückgabe und damit mit einem Blutverlust des Patienten einhergeht. Das erfindungsgemäße Verfahren vermeidet diese Nachteile in einigen Ausführungsformen.
- Verringerte Anämie bei Dialysepatienten
- Verringerter Bedarf von Medikamenten, welche die Blutbildung unterstützen (z.B. Erythropoetin)
- Steigerung des Wohlbefindens des Patienten
- Verringerung der "Effektiven Behandlungszeit" (Dialysieren) während der Reinfusion und entsprechende Verkürzung der Dialysezeit (mit entsprechender Kosteneinsparung) und/oder verbesserte Clearance

Die vorliegende Erfindung mindert in bestimmten Ausführungsformen vorteilhaft die während der Reinfusion zusammen mit dem Blut infundierte Substituatmenge.

So kann die vorliegende Erfindung in bestimmten erfindungsgemäßen Ausführungsformen zu einer Verbesserung der Effektivität der arterio-venösen-Blutrückgabe, also der simultanene Rückreinfusion sowohl über die arterielle als auch über die venöse Patientenkonnektion beitragen.

Da das erfindungsgemäße Verfahren direkt nach Beenden einer Blutbehandlungssitzung durchgeführt werden kann, ist es einfach und leicht durchzuführen und erfordert keine technisch aufwändigen, zeit- und/oder kostenintensiven Schritte.

Das erfindungsgemäße Verfahren kann in vorteilhafter Weise vorzugsweise mit dem bei einer Blutbehandlung ohnehin eingesetzten oder vorliegenden Substituat oder der bei einer Blutbehandlung ohnehin eingesetzten oder vorliegenden Flüssigkeit, wie beispielsweise einer isotonen Kochsalzlösung, z. B. einer 0,9%igen NaCl-Lösung, durchgeführt werden. Dies trägt wiederum in vorteilhafter Weise zu einer Kosten- und Zeitersparnis bei.

Ferner kann das erfindungsgemäße Verfahren ein Entfernen von Blut aus dem arteriellen Abschnitt des extrakorporalen Blutkreislaufs und insbesondere aus der arteriellen Konnektionsnadel und ein Rückführen desselben in das Gefäßsystem des Patienten ermöglichen. Es kann somit in vorteilhafter Weise jener Schritt von bekannten Rückgabeverfahren umgangen werden, bei welchem in der arteriellen Konnektionsnadel befindliches Blut mit Hilfe z. B. einer mit Kochsalzlösung aufgezogenen Spritze retrograd herausgedrückt wird.

Das erfindungsgemäße Verfahren kann somit den Vorteil bieten, das im Leitungsinneren eines extrakorporalen Blutkreislaufs nach dessen Verwendung bei einer Blutbehandlung vorhandene Blut im Wesentlichen vollständig für den Patienten zurückzugewinnen.

Die erfindungsgemäße Vorgehensweise kann sicherstellen, dass beim Entleeren keine Luft in das Gefäßsystem des Patienten eintritt. Ferner kann es in einigen Ausführungsformen bei diesem Verfahren zu keiner Schaumbildung im Bereich eines im extrakorporalen Blutkreislauf vorhandenen Blutfilters kommen, was ein Entleeren des Bluts aus dem extrakorporalen Blutkreislauf erschweren würde. Im Blutfilter oder im extrakorporalen Blutkreislauf verbleibendes Blut bildet aber wiederum ein Kontaminationsrisiko.

Im Folgenden wird das erfindungsgemäße Verfahren anhand bevorzugter Ausführungsformen desselben unter Bezugnahme auf die angehängte Zeichnung beschrieben. In der Zeichnung gilt:

**Fig. 1**   zeigt schematisch vereinfacht die Durchführung eines Verfahrens aus dem Stand der Technik mittels einer bekannten medizinischen Behandlungseinrichtung;

**Fig. 2**   zeigt schematisch vereinfacht eine erfindungsgemäße medizinische Behandlungsvorrichtung bei der Durchführung des erfindungsgemäßen Verfahrens in einer beispielhaften Ausführungsform; und

**Fig. 3**   zeigt ein weiteres Verfahren aus dem Stand der Technik, durchgeführt mittels in einer bekannten medizinischen Behandlungseinrichtung; und

**Fig. 4**   zeigt den Vergleich zwischen einer herkömmlichen Blutrückgabe mit NaCl und einer Blutrückgabe mittels des erfindungsgemäßen Verfahrens.

Die Erfindung wird durch die Merkmale der unabhängigen Ansprüche definiert. Die im Rahmen dieser Beschreibung offenbarten Verfahren fallen als solche nicht unter den beanspruchten Gegenstand der Ansprüche.

Nach Beendigung einer Blutbehandlungssitzung wird typischerweise das im extrakorporalen Blutkreislauf (und insbesondere im Blutfilter) vorhandene Blut und/oder Blutgemisch an den Patienten zurückgeführt. Dies geschieht üblicherweise durch Einleiten von Substituat in den extrakorporalen Blutkreislauf, wodurch das dort vorhandene Blut stromab verdrängt wird und somit dem Patienten reinfundiert wird, z.B. über den venösen Patientenanschluss.

Dabei entsteht im Stand der Technik in der Regel ein unscharfer Übergangsbereich von Blut zu Substituat, in welchem sich Blut mit Substituat vermischt. Das in Strömungsrichtung hinter dem Blutfilter (post-Filter) befindliche Blut-Substituatgemisch enthält somit regelmäßig Substituat, und für das Freispülen des extrakorporalen Blutkreislaufs bis hin zum venösen Patientenanschluss wird dabei unerwünscht viel Substituat benötigt. Ein Anteil dieses Substituats wird überdies dem Patienten zugeführt, was insbesondere bei Dialysepatienten nach Abschluss der Blutbehandlung in der Regel unerwünscht ist.

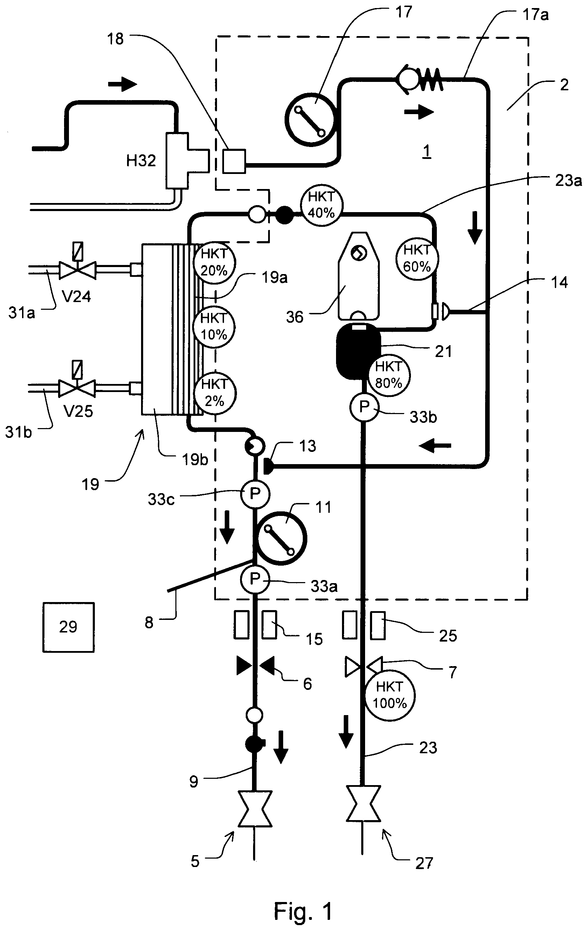

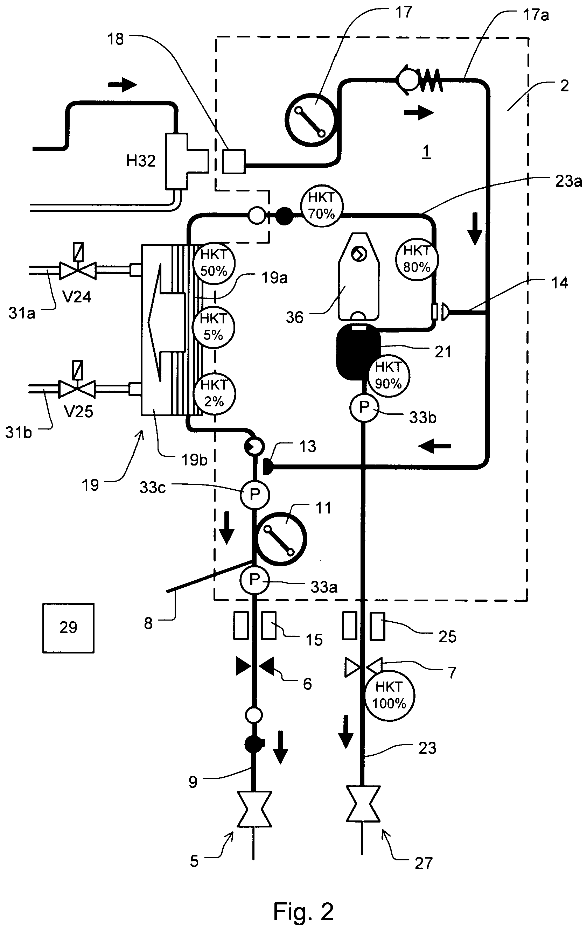

In Fig. 1 wird ein herkömmliches Verfahren und in Fig. 2 das erfindungsgemäße Verfahren zum Entfernen von Blut und/oder eines Blutgemischs aus einem zur Blutbehandlung eines Patienten verwendeten Blutfilter 19 nach Beendigung der Blutbehandlungssitzung in einer exemplarischen Ausführungsform dargestellt. Dabei ist jeweils ein Beispiel mit simultaner Reinfusion (auch als "online closed circuit" bezeichnet) und Restblutverteilung nach Förderung der halben herkömmlichen Reinfusionsmenge (z. B. rund 200 ml) schematisch und in Momentaufnahme gezeigt.

Fig. 1 zeigt einen extrakorporalen Blutkreislauf 1, welcher mittels Double-Needle-Zugang mit dem Gefäßsystem des nicht dargestellten Patienten verbunden oder verbindbar ist. Der Blutkreislauf 1 liegt optional in Abschnitten hiervon in oder auf einer Blutkassette 2 vor. Er ist mit einer Behandlungsvorrichtung 4 verbunden. Die Steuerung oder Regelung der Behandlungsvorrichtung 4 kann mittels einer Steuerungs- oder Regelungseinrichtung 29 erfolgen.

Der extrakorporale Blutkreislauf 1 weist eine arterielle Patientenschlauchklemme 6 und eine arterielle Konnektionsnadel 5 (als Beispiel einer Zugangseinrichtung) eines arteriellen Abschnitts 9 oder einer arteriellen Patientenleitung oder Blutleitung 9 auf. Der extrakorporale Blutkreislauf 1 weist ferner eine venöse Patientenschlauchklemme 7 und eine venöse Konnektionsnadel 27 (als Beispiel einer weiteren oder zweiten Zugangseinrichtung) eines venösen Abschnitts 23 oder einer venösen Patientenleitung oder Blutleitung 23 auf.

Eine Blutpumpe 11 ist im arteriellen Abschnitt 9 vorgesehen, eine Substituatpumpe 17 ist mit einer Substituatleitung 17a verbunden. Die Substituatleitung 17a kann mittels eines, vorzugsweise automatischen, optionalen, hier als nicht verbunden gezeigten Substituatports 18 mit einer Substituatquelle verbunden werden. Mittels der Substituatpumpe 17 kann Substituat per Prädilution oder per Postdilution über zugehörige Leitungen 13 bzw. 14 in Leitungsabschnitte, beispielsweise in den arteriellen Abschnitt 9 bzw. in einen venösen Abschnitt 23a (zwischen der Blutkammer 19a und einer optionalen Single-Needle-Kammer 36) des extrakorporalen Blutkreislaufs 1 eingebracht werden.

In den Blutkreislauf 1 ist der Blutfilter 19 eingeschaltet. Dieser weist die mit dem arteriellen Abschnitt 9 und mit dem venösen Abschnitt 23 verbundene Blutkammer 19a auf. Eine Dialysatkammer 19b des Blutfilters 19 ist mit einer zur Dialysatkammer 19b führenden Dialysierflüssigkeitszulaufleitung 31a und einer von der Dialysatkammer 19b fortführenden Dialysatablaufleitung 31b verbunden.

Die Dialysierflüssigkeitszulaufleitung 31a weist optional ein Ventil V24 auf, mittels welchem der Fluss innerhalb der Dialysierflüssigkeitszulaufleitung 31a unterbunden werden kann. Die Dialysatablaufleitung 31b weist optional ein Ventil V25 auf, mittels welchem der Fluss innerhalb der Dialysatablaufleitung 31b unterbunden werden kann.

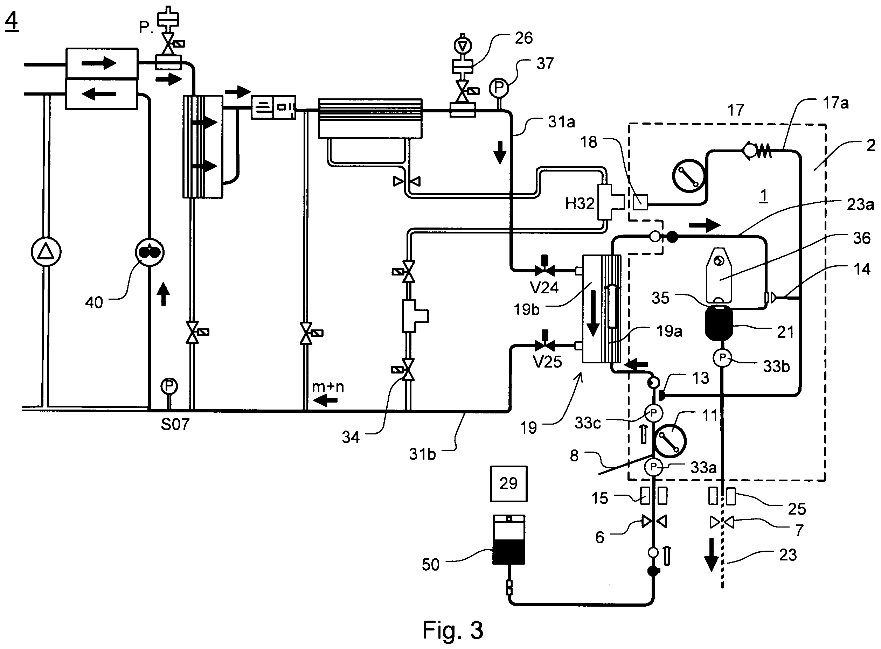

Die Dialysierflüssigkeitszulaufleitung 31a ist ferner optional mittels eines weiteren, maschineninternen Ventils mit einer Druckluftquelle 26 verbunden (hier nicht gezeigt, siehe aber Fig. 3). Die Druckluftquelle 26 kann Bestandteil der Behandlungsvorrichtung 4 sein oder getrennt hiervon vorliegen. Stromab der Druckluftquelle 26 kann ein Drucksensor 37 (hier nicht gezeigt, siehe aber Fig. 3) vorgesehen sein.

Die Anordnung der Fig. 1 umfasst einen optionalen, arteriellen Detektor 15 zum Detektieren von Luft und/oder Blut. Die Anordnung der Fig. 1 umfasst ferner einen, zwei oder mehr Drucksensor(en) 33a, 33b, 33c, z. B. an den in Fig. 1 und 2 gezeigten Stellen.

Zum Leeren der Blutkammer 19a des Blutfilters 19 von Blut nach Ende der Behandlung kann, wie in Fig. 1 gezeigt, Substituat mittels der Substituatpumpe 17 über die Zugabestelle 13 in Prädilution dem Blutkreislauf 1 und der Blutkammer 19a zugeführt werden.

Alternativ, oder ergänzend, kann das Substituat nicht oder nicht nur mittels der Substituatpumpe 17 sondern (auch oder allein) durch Betreiben der Blutpumpe 11 eingebracht werden.

Dazu wird z.B. die arterielle Patientenschlauchklemme 6 geschlossen und Substituat über eine Zuleitung 8 aus einem Vorratsbehälter für das Substituat in den extrakorporalen Blutkreislauf 1 eingebracht.

Der so entstandene Substituat-Blut-Inhalt wird durch Betreiben der Blutpumpe 11 und/oder der Substituatpumpe 17 entlang des Leitungsinneren des extrakorporalen Blutkreislaufs 1 gefördert und durch den Blutfilter 19, eine venöse Luftabscheidekammer 21 und den venösen Abschnitt 23 des extrakorporalen Blutkreislaufs 1 gedrückt bzw. gefördert, um so das Blut aus dem extrakorporalen Blutkreislauf 1 in Richtung zur venösen Konnektionsnadel 27 aus dem Blutfilter 19 zu entfernen.

Im venösen Abschnitt 23 des extrakorporalen Blutkreislaufs 1 ist optional ein venöser Substituat-Blut-Detektor 25 als ein Beispiel einer Erfassungseinrichtung angeordnet, der das Auftreten von Substituat an einer vorbestimmten Position des Leitungsinneren des extrakorporalen Blutkreislaufs 1 erkennt. Die Blutpumpe 11 und/oder die Substituatpumpe 17 fördert den Substituat-Blut-Inhalt optional solange weiter, bis Blut, das im venösen Abschnitt 23 des extrakorporalen Blutkreislaufs 1, vorgelegen hat, aus selbigem entfernt und über die venöse Konnektionsnadel 27 in das Gefäßsystem des Patienten rückgeführt worden ist und/oder bis am venösen Substituat-Blut-Detektor 25 das Auftreten von Substituat (oder das Abfallen des Hämatokrits im Leitungsinneren, z.B. bis auf 2%) im Leitungsinneren erfasst wird. Die Förderleistung aller Pumpen kann nun beendet werden. Es kann ein optisches und/oder akustisches Signal ausgegeben werden.

**Fig. 1** zeigt eine Restblutverteilung nach Förderung der halben Menge an Reinfusionsfluid, das zum Entfernen von Blut aus dem Blutschlauchsatz 1 herkömmlich benötigt wird. Der Hämatokrit HKT am venösen Substituat-Blut-Detektor 25 beträgt zu dem in Fig. 1 dargestellten Zeitpunkt 100% des ursprünglichen, unmittelbar vor Beendigung des Blutbehandlungsverfahrens im extrakorporalen Blutkreislauf 1 vorhandenen HKT-Werts. Der Hämatokrit HKT entspricht am venösen Substituat-Blut-Detektor 25 somit dem ursprünglichen, unmittelbar vor Beendigung des Blutbehandlungsverfahrens im extrakorporalen Blutkreislauf 1 vorhandenen HKT-Wert. Der Hämatokrit HKT ist daher am venösen Substituat-Blut-Detektor 25 in Fig. 1 als "HKT 100%" angegeben. Es ist wichtig zu wissen, dass alle Prozentangaben zum HKT in Fig. 1 und Fig. 2 relative Angaben sind: die an der angegebenen Stelle des Blutschlauchkreislaufs 1 angegebenen Prozentzahlen geben an, welchen prozentualen Anteil des zu Behandlungsende im Leitungsinneren vorliegenden HKT der an den bezeichneten Stellen jeweils messbare HKT beträgt.

Der Hämatokrit HKT des im extrakorporalen Blutkreislauf 1 vorliegenden Bluts wird in Fig. 1 an verschiedenen Stellen mit HKT und einem Prozentwert beschrieben. Der Prozentwert beschreibt, in welchem Verhältnis der aktuelle Hämatokrit HKT an der gezeigten Stelle zu dem ursprünglichen, unmittelbar vor Beendigung des Blutbehandlungsverfahrens im extrakorporalen Blutkreislauf 1 vorhandenen Hämatokrit steht. Wenn beispielsweise der Hämatokrit HKT im extrakorporalen Blutkreislauf 1 vor Beendigung der Blutbehandlung 42% betrug, dann bedeutet "HKT 100%", dass der Hämatokrit HKT weiterhin bei 42% liegt.

Durch Infusion von Substituat mittels der Substituatpumpe 17 über die Zugabestelle 13 für Prädilution entsteht am Übergang von Substituat zu Blut eine Vermischung im extrakorporalen Blutkreislauf 1, insbesondere im Blutfilter 19, die Einfluss auf den messbaren HKT hat.

In Fig. 1 beträgt der Hämatokrit HKT am Eingang der Blutkammer 19a des Blutfilters 19 2% des ursprünglichen Wertes. Der Hämatokrit HKT steigt über die Blutkammer 19a hinweg an - zunächst auf 10% und dann am venösen Ende der Blutkammer 19a auf 20% des ursprünglich messbaren Werts. Im venösen Abschnitt 23 steigt der Hämatokrit HKT weiter an auf zunächst 40% kurz hinter dem Blutfilter 19, dann auf 60% im Abschnitt 23a, auf 80% hinter der Luftabscheidekammer 21 bis hin zu 100% am venösen Substituat-Blut-Detektor 25 (in Fig. 1 wie in Fig. 2 angegebene Prozentwerte beziehen sich auf das Verhältnis des aktuell vorliegenden Hämatokrits HKT zum ursprünglichen Hämatokrit HKT, s.o., und sind somit relative Werte). Hier wird deutlich, dass die Vermischung von Substituat und Blut im Stand der Technik über eine längere Strecke hinweg stattfindet, so dass zur vollständigen oder fast vollständigen Reinfusion des Bluts ein erhebliches Volumen an Substituat infundiert werden muss.

Das in **Fig. 2** in Momentaufnahme dargestellte erfindungsgemäße Verfahren zeigt - wie Fig. 1 - eine Restblutverteilung nach Förderung der halben herkömmlichen Reinfusionsmenge. Im Unterschied zu Fig. 1 besteht jedoch hier eine Druckdifferenz im Blutfilter 19 bei einem niedrigeren Druck in der Dialysatkammer 19b und einem höheren Druck in der Blutkammer 19a. Die Druckdifferenz kann dabei beispielsweise hervorgerufen werden durch das Erzeugen eines absoluten oder relativen Unterdrucks in der Dialysatkammer 19b des Blutfilters 19, beispielsweise durch Entfernen von Flüssigkeit aus der Dialysatkammer 19b über das Ventil V25 mittels einer Ultrafiltrationspumpe (UF-Pumpe 40, nicht gezeigt, s. Fig. 3). Die Druckdifferenz kann alternativ oder zusätzlich erzeugt werden durch einen Flusswiderstand stromab der Blutkammer 19a, beispielsweise im venösen Abschnitt 23a, wenn zugleich Dialysierflüssigkeit in die Blutkammer 19a eingebracht wird, beispielsweise mittels der Substituatpumpe 17 und/oder der Blutpumpe 11.

Mittels der Druckdifferenz wird Flüssigkeit aus dem extrakorporalen Blutkreislauf 1 entfernt (s. Pfeil im Blutfilter 19). Dies führt dazu, dass der Hämatokrit HKT im extrakorporalen Blutkreislauf 1 während der Blutrückgabe grundsätzlich oder streckenweise höher ist als in Fig. 1. Dies ist zu sehen am Blutfilter 19, an dessen Eingang, genau wie in Fig. 1, ein Hämatokrit HKT von 2% (des ursprünglichen Werts) festgestellt werden kann. Am Ausgang des Filters 19 beträgt der Hämatokrit HKT jedoch bereits 50% gegenüber 20% (jeweils des ursprünglichen Werts) des in Fig. 1 gezeigten Verfahrens. Dies ist darauf zurückzuführen, dass im Blutfilter 19 dem Blut-Substituat-Gemisch Wasser entzogen wurde.

An den Stellen, an denen der Hämatokrit HKT in Fig. 1 40%, 60% und 80% beträgt, liegt dieser in Fig. 2 bei 70%, 80% beziehungsweise 90%. Der Übergang von Blut zu Substituat ist somit schärfer als im herkömmlichen Verfahren der Fig. 1. Für das Freispülen bis zum venösen Patientenanschluss wird weniger Substituat benötigt. Zudem enthält das stromab des Filters 19 befindliche Blut-Substituatgemisch vergleichsweise weniger Substituat.

**Fig. 3** zeigt ein beispielhaftes Geräteverhalten während einer aus dem Stand der Technik bekannten Blutrückgabe. Als Quelle des Substituats, mit dem das Blut aus dem extrakorporalen Blutkreislauf 1 verdrängt wird, dient ein Beutel 50 mit physiologischer Kochsalzlösung, der am arteriellen Abschnitt 9 angebracht wird. Anders als in Fig. 1 und 2 wird das Substituat mit Hilfe der Blutpumpe 11 in den Blutfilter 19 gefördert.

Dabei wird im Blutfilter 19 kein Patientenblut mehr gereinigt, die Dialysatkammer 19b und die Membran des Blutfilters 19 wird nicht weiter durchströmt, so dass die Wassermenge (Plasma) im Patientenblut nicht weiter verringert wird. Somit erreicht die Flussmenge der Blutpumpe 11 den Patienten (die Flussrate beträgt z.B. 30 bis 200 ml/min). Die Ventile V24 und V25 sind beide geschlossen, und die UF-Pumpe 40 ist ausgeschaltet. Die arterielle Blutpumpe 11 fördert NaCl-Lösung in den extrakorporalen Blutkreislauf 1. Die Flussrate beträgt dabei z.B. 30 bis 200 ml/min.

Das Geräteverhalten in einer erfindungsgemäßen beispielhaften Ausführungsform kann während der Blutrückgabe ebenfalls anhand von Fig. 3 illustriert werden. Die Dialysatkammer 19b wird in einer Ausführungsform optional nicht länger durchströmt, das heißt es findet - außer über die Membran - kein Fluss in die Dialysatkammer hinein statt. Die Wassermenge (der Plasma-Anteil) des Patientenbluts wird weiter verringert. Dabei ist das Ventil V24 geschlossen und das Ventil V25 geöffnet. Die UF-Pumpe 40 ist eingeschaltet und pumpt mit z.B. 1 bis 80 ml/min, um einen Unterdruck in der Dialysatkammer 19b zu erzeugen und hierdurch über die Membran hinweg Wasser aus der Blutkammer 19a zu entfernen. Die arterielle Blutpumpe 11 fördert hier exemplarisch NaCl-Lösung aus dem Beutel am arteriellen Abschnitt 9 in den extrakorporalen Blutkreislauf 1 mit einer Flussrate von z.B. 30 bis 280 ml/min.

Die Flussrate innerhalb des extrakorporalen Blutkreislaufs 1 über die Membran des Filters 19 hinweg zur Behandlungsvorrichtung 4 hin entspricht in diesem Beispiel der Förderrate der UF-Pumpe 40. Die Flussmenge, die den Patienten erreicht, ist in diesem Beispiel die von der arteriellen Blutpumpe geförderte Flussmenge abzüglich der von der UF-Pumpe geförderten Flussmenge. Die Flussrate in das Gefäßsystem des Patienten hinein (Summe von arteriell und venös) beträgt hier z.B. 30 bis 200 ml/min.

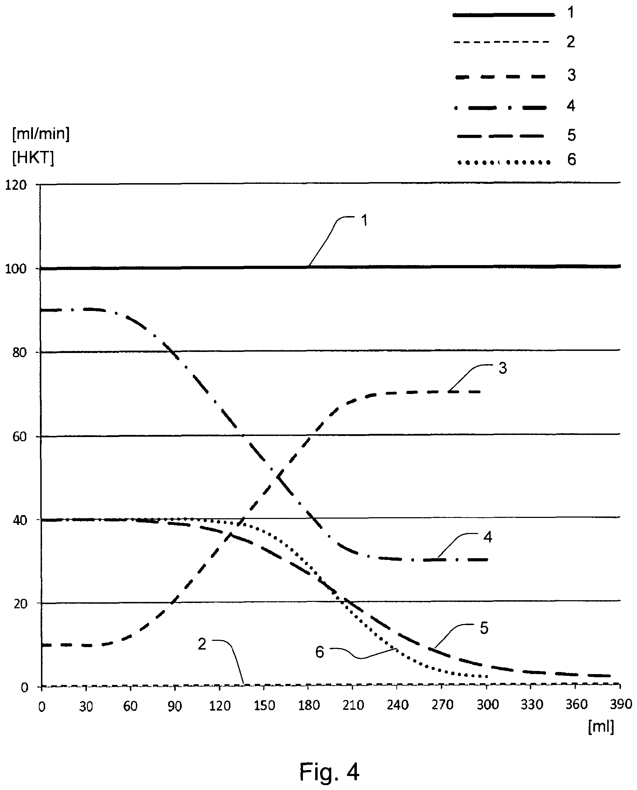

**Fig. 4** zeigt einen Vergleich zwischen einer Blutrückgabe aus dem Stand der Technik und einer exemplarischen Ausführungsform des erfindungsgemäßen Verfahrens.

In diesem Beispiel wird durch das erfindungsgemäße Verfahren die notwendige Menge an Substituat vorteilhaft von den üblichen 390 ml auf nur 300 ml verringert.

Die durchgezogene Linie (Linie 1) bezeichnet die Flussrate, die am Eingang des Blutfilters 19 vorliegt. Diese Flussrate ist sowohl im Stand der Technik als auch im beispielhaften erfindungsgemäßen Verfahren während der gesamten Blutrückgabe optional unverändert und kann bei 100 ml/min liegen.

Im Stand der Technik liegt während der Blutrückgabe die Förderrate der UF-Pumpe bei 0 ml/min, wie an der dünnen, kurz gestrichelten Linie (Linie 2) zu sehen ist. Es findet kein Fluss über die Filtermembran statt. Daher ist die Flussrate am Eingang des Filters 19 (Linie 1) hier gleich der Flussrate am Ausgang des Filters 19.

Daraus ergibt sich im Stand der Technik (Linie 5, lang gestrichelt) ein absoluter Hämatokrit HKT am venösen Patientenanschluss, in diesem Fall am venösen Substituat-Blut-Detektor 25, der zu Beginn der Blutrückgabe 40% beträgt. Durch die gewünschte Verdrängung des Bluts mittels Substituats nimmt der Hämatokrit HKT während der Blutrückgabe am Detektor 25 ab, bis er dort einen vorbestimmten Wert von z.B. 2% erreicht. Um diesen Wert von 2% zu erreichen, müssen im Stand der Technik 390 ml physiologische Kochsalzlösung in den extrakorporalen Blutkreislauf eingebracht werden (s. x-Achse des Diagramms der Fig. 4).

In der in Fig. 4 beispielhaft dargestellten Ausführungsform des erfindungsgemäßen Verfahrens ist der Fluss am Filterausgang (Linie 4, strichpunktiert) niedriger als der Fluss am Filtereingang (Linie 1), da die UF-Pumpe 40 Wasser aus der Dialysatkammer 19b entfernt. Die Förderrate der UF-Pumpe 40 ist mit der dicken, kurz gestrichelten Linie (Linie 3) dargestellt. Hier wird deutlich, dass die Förderrate der UF-Pumpe 40 über die Blutrückgabe hinweg ansteigt (Linie 3) während als Folge die Flussrate am Filterausgang (Linie 4) absinkt. Dem Blut-Substituatgemisch wird im Blutfilter 19 durch die UF-Pumpe 40 Wasser entzogen, wodurch der Hämatokrit HKT am venösen Patientenanschluss 27 schon früher, nämlich nach Infusion von 300 ml Substituat, auf den vorbestimmten Wert (z.B. 2%) absinkt (Linie 6, gestrichelt). Für eine fast vollständige Reinfusion des im extrakorporalen Blutkreislauf 1 enthaltenen Blutvolumens wird somit erfindungsgemäß weniger Substituat infundiert als im Stand der Technik.

Die vorliegende Erfindung ist nicht auf die vorstehend beschriebenen Ausführungsformen beschränkt, diese dienen lediglich der Veranschaulichung. Die Erfindung wird vielmehr durch die Merkmale der unabhängigen Ansprüche definiert.

**Bezugszeichenliste**

| | |
|---|---|
| 1 | extrakorporaler Blutkreislauf |
| 2 | Blutkassette |
| 4 | Behandlungsvorrichtung, Blutbehandlungsvorrichtung |
| 5 | Zugangseinrichtung, beispielsweise arterielle Konnektionsnadel |
| 6 | arterielle Patientenschlauchklemme |
| 7 | venöse Patientenschlauchklemme |
| 8 | Zuleitung |
| 9 | arterieller Abschnitt oder arterielle Blutleitung oder arterielle Patientenleitung |
| 11 | Blutpumpe |
| 13 | Zugabestelle für Substituat (Prädilution) |
| 14 | Zugabestelle für Substituat (Postdilution) |
| 15 | arterieller Luft-Blut-Detektor |
| 17 | zweite Fördereinrichtung, beispielsweise eine Substituatpumpe |
| 17a | Substituatleitung |
| 18 | automatischer Substituatport |
| 19 | Blutfilter, Filter |
| 19a | Blutkammer |
| 19b | Dialysatkammer |
| 21 | venöse Luftabscheidekammer |
| 23 | venöser Abschnitt oder venöse Blutleitung |
| 23a | venöser Abschnitt |
| 25 | venöser Substituat-Blut-Detektor |
| 26 | Druckluftquelle |
| 27 | Zugangseinrichtung, beispielsweise venöse Konnektionsnadel, venöser Patientenanschluss |
| 29 | Steuerungs- oder Regelungseinrichtung |
| 31a | Dialysierflüssigkeitszulaufleitung |

| | |
|---|---|
| 31b | Dialysatablaufleitung |
| 33a, b, c | Drucksensoren |
| 35 | Single-Needle-Ventil |
| 36 | Single-Needle-Kammer |
| 37 | Drucksensor |
| 40 | Ultrafiltrationspumpe (UF-Pumpe) |
| V24 | Ventil |
| V25 | Ventil |
| 50 | Beutel |
| HKT | Hämatokrit |

**Patentansprüche**

1. Steuerungs- und/oder Regelungseinrichtung (29), konfiguriert zum Steuern oder Regeln einer medizinischen Blutbehandlungsvorrichtung (4) zum Durchführen eines Verfahrens zum Entfernen von Blut und/oder eines Blutgemischs aus einem zur Blutbehandlung eines Patienten verwendeten extrakorporalen Blutkreislauf (1) mit einem Blutfilter (19) nach Beendigung einer Blutbehandlungssitzung während eines Reinfusionsverfahrens, wobei der Blutfilter (19) eine Blutkammer (19a) und eine Dialysatkammer (19b) aufweist, zwischen welchen eine Membran angeordnet ist, wobei die Blutkammer (19a) zur Blutbehandlung mit einer zur Blutkammer (19a) führenden arteriellen Blutleitung (9) und einer von der Blutkammer (19a) wegführenden venösen Blutleitung (23) sowie einer zur Dialysatkammer (19b) führenden Dialysierflüssigkeitszulaufleitung (31a) und einer von der Dialysatkammer (19b) wegführenden Dialysatablaufleitung (31b) verbunden ist,
wobei das Verfahren die folgenden Schritte umfasst:

   - Verdrängen des Bluts und/oder des Blutgemischs aus der Blutkammer (19a) durch Einbringen von Substituat in die arterielle Blutleitung (9) unter Entstehung eines Blut-Substituatgemisches; und **gekennzeichnet durch** den Schritt
   - Erzeugen einer Druckdifferenz im Blutfilter (19) mit einem niedrigeren Druck in der Dialysatkammer (19b) und einem höheren Druck in der Blutkammer (19a) während des Reinfusionsvorgangs zum Entziehen von Wasser aus dem Blut-Substituatgemisch über den Blutfilter (19).

2. Steuerungs- und/oder Regelungseinrichtung (29) nach Anspruch 1, wobei die Druckdifferenz, zumindest teilweise, durch mindestens eine Pumpe, insbesondere eine Ultrafiltrationspumpe (40), eine Substituatpumpe (17) und/oder eine Blutpumpe (11) erzeugt wird.

3. Steuerungs- und/oder Regelungseinrichtung (29) nach Anspruch 2, wobei die Ultrafiltrationspumpe (40), zumindest zeitweise, gleichzeitig mit der Substituatpumpe (17) und/oder der Blutpumpe (11) betrieben wird.

4. Steuerungs- und/oder Regelungseinrichtung (29) nach einem der vorhergehenden Ansprüche, wobei die Flussrate ($Q_{BP}$) der Blutpumpe (11) und/oder die Flussrate ($Q_{substituatpumpe}$) der Substituatpumpe (17) zwischen 30 bis 280 ml/min beträgt.

5. Steuerungs- und/oder Regelungseinrichtung (29) nach einem der vorhergehenden Ansprüche, wobei das Verhältnis ($Q_{UF}/Q_{BP}$) der Flussraten von Ultrafiltrationspumpe (40) zu Blutpumpe (11) und/oder das Verhältnis ($Q_{UF}/Q_{Substituatpumpe}$) der Flussraten von Ultrafiltrationspumpe (40) zu Substituatpumpe (17) in einem Wertebereich von 0,01 bis 0,8 liegt.

6. Steuerungs- und/oder Regelungseinrichtung (29) nach einem der vorhergehenden Ansprüche, wobei das Verhältnis ($Q_{UF}/Q_{BP}$) und/oder das Verhältnis ($Q_{UF}/Q_{Substituatpumpe}$) während der Ausführung des Verfahrens in Abhängigkeit des bisher oder seit Beginn des Verfahrens geförderten Substituatvolumens ansteigt.

7. Steuerungs- und/oder Regelungseinrichtung (29) nach einem der vorhergehenden Ansprüche, wobei das Verhältnis ($Q_{UF}/Q_{BP}$) und/oder das Verhältnis ($Q_{UF}/Q_{Substituatpumpe}$) während der Ausführung des Verfahrens variiert wird, um den Hämatokrit (HKT) in der venösen Blutleitung (23) auf einen vorbestimmten Wert zu steuern oder zu regeln.

8. Steuerungs- und/oder Regelungseinrichtung (29) nach einem der vorhergehenden Ansprüche, wobei das Verhältnis ($Q_{UF}/Q_{BP}$) und/oder das Verhältnis ($Q_{UF}/Q_{substituatpumpe}$) in Abhängigkeit des mittels einer Erfassungseinrichtung, insbesondere mittels eines venösen Substituat-Blut-Detektors (25), bestimmten Hämatokrits (HKT) in der venösen Blutleitung (23) und in Abhängigkeit eines vorbestimmten, einzusparenden Substituatvolumens geregelt wird.

9. Medizinische Behandlungsvorrichtung (4) mit

- optional wenigstens einem extrakorporalen Blutkreislauf (1) mit einem Leitungsinneren;
- wenigstens einer an oder im extrakorporalen Blutkreislauf (1) angeordneten Blutpumpe (11) zum Fördern von Blut innerhalb des Leitungsinneren des extrakorporalen Blutkreislaufs (1);
**gekennzeichnet durch**
- wenigstens eine Steuerungs- und/oder Regelungseinrichtung (29) nach einem der Ansprüche 1 bis 8.

10. Medizinische Behandlungsvorrichtung (4) nach Anspruch 9, welche wenigstens eine Pumpe zum Erzeugen einer Druckdifferenz im Blutfilter (19), mit einem niedrigeren Druck in der Dialysatkammer (19b) und einem höheren Druck in der Blutkammer (19a), aufweist oder hiermit, vorzugsweise in Fluidkommunikation, verbunden ist.

11. Digitales Speichermedium, insbesondere in Form einer Diskette, CD oder DVD oder EPROM, mit elektronisch auslesbaren Steuersignalen, konfiguriert, um derart mit einem programmierbaren Computersystem einer medizinischen Blutbehandlungsvorrichtung zusammenzuwirken, dass die in einem der Ansprüche 1 bis 8 genannten maschinellen Schritte veranlasst werden.

12. Computerprogramm-Produkt mit einem auf einem maschinenlesbaren Träger gespeicherten Programmcode zur Veranlassung der in einem der Ansprüche 1 bis 8 genannten maschinellen Schritte, wenn das Computerprogramm-Produkt auf einem Rechner einer medizinischen Blutbehandlungsvorrichtung abläuft.

13. Computerprogramm mit einem Programmcode zur Veranlassung der in einem der Ansprüche 1 bis 8 genannten maschinellen Schritte, wenn das Computerprogramm auf einem Computer einer medizinischen Blutbehandlungsvorrichtung abläuft.

## Claims

1. A control and/or a regulating device (29), configured for controlling or regulating a medical blood treatment apparatus (4) for performing a method to remove blood and/or a blood mixture from an extracorporeal blood circuit (1) used for blood treatment of a patient with a blood filter (19) after completion of a blood treatment session during a reinfusion process, wherein the blood filter (19) comprises a blood chamber (19a) and a dialysate chamber (19b), between which a membrane is arranged, wherein for blood treatment, the blood chamber (19a) is connected to an arterial blood line (9) leading to the blood chamber (19a) and to a venous blood line (23) leading away from the blood chamber (19a) as well as to a dialysis fluid inlet line (31a) leading to the dialysate chamber (19b) and to a dialysate outlet line (31b) leading away from the dialysate chamber (19b), the method comprising the following step of:

- displacing the blood and/or the blood mixture from the blood chamber (19a) by introducing substituate into the arterial blood line (9) under formation of a blood-substituate mixture; and

**characterized by** the step of:

- generating a pressure difference in the blood filter (19) with a lower pressure in the dialysate chamber (19b) and a higher pressure in the blood chamber (19a) during the reinfusion process for removing water from the blood-substituate mixture via the blood filter (19).

2. The control and/or regulating device (29) according to claim 1, wherein the pressure difference is, at least partially, generated by at least one pump, in particular an ultrafiltration pump (40), a substituate pump (17) and/or a blood pump (11).

3. The control and/or regulating device (29) according to claim 2, wherein the ultrafiltration pump (40) is, at least temporarily, activated simultaneously with the substituate pump (17) and/or the blood pump (11).

4. The control and/or regulating device (29) according to anyone of the preceding claims, wherein the flow rate ($Q_{BP}$) of the blood pump (11) and/or the flow rate ($Q_{Substituatpumpe}$) of the substituate pump (17) is between 30 and 280 ml/min.

5. The control and/or regulating device (29) according to anyone of the preceding claims, wherein the flow rate ratio ($Q_{UF}/Q_{BP}$) of ultrafiltration pump (40) to blood pump (11) and/or the flow rate ratio ($Q_{UF}/Q_{Substituatpumpe}$) of ultrafiltration pump (40) to substitute pump (17) is/are in a value range of 0.01 to 0.8.

6. The control and/or regulating device (29) according to anyone of the preceding claims, wherein the ratio ($Q_{UF}/Q_{BP}$) and/or the ratio ($Q_{UF}/Q_{Substituatpumpe}$) increase (s) during the execution of the process depending on the substitute volume conveyed so far or since the beginning of the process.

7. The control and/or regulating device (29) according to anyone of the preceding claims, wherein the ratio ($Q_{UF}/Q_{BP}$) and/or the ratio ($Q_{UF}/Q_{Substituatpumpe}$) is/are varied during the execution of the process in order to control or regulate the hematocrit (HKT) in the venous blood line (23) to a predetermined value.

8. The control and/or regulating device (29) according to anyone of the preceding claims, wherein the ratio ($Q_{UF}/Q_{BP}$) and/or the ratio ($Q_{UF}/Q_{Substituatpumpe}$) is/are regulated depending on the hematocrit (HTK) in the venous blood line (23), which is determined by means of a detection device, in particular by means of a venous substituate-blood detector (25), and depending on a predetermined substitute volume to be saved.

9. A medical blood treatment apparatus (4) having

   - optionally at least one extracorporeal blood circuit (1) with a line interior;
   - at least one blood pump (11) arranged on or in the extracorporeal blood circuit (1) for conveying blood within the line interior of the extracorporeal blood circuit (1);
   **characterized by**
   - at least one control and/or regulating device (29) according to anyone of claims 1 to 8.

10. The medical blood treatment apparatus (4) according claim 9, which comprises, or is connected to, preferably in fluid communication, at least one pump for generating a pressure difference in the blood filter (19), with a lower pressure in the dialysate chamber (19b) and a higher pressure in the blood chamber (19a).

11. A digital storage medium, in particular in the form of a floppy disk, CD or DVD or EPROM, with electronically readable control signals, configured for interacting with a programmable computer system of a medical blood treatment apparatus such that the mechanical steps mentioned in anyone of claims 1 to 8 are prompted.

12. A computer program product having a program code saved on a machine-readable medium for prompting the mechanical steps mentioned in anyone of claims 1 to 8 when the computer program product runs on a computer of a medical blood treatment apparatus.

13. A computer program having a program code for prompting the mechanical steps mentioned in anyone of claims 1 to 8 when the computer program runs on a computer of a medical blood treatment apparatus.


**Revendications**

1. Un dispositif de commande et/ou de régulation (29), configuré pour commander ou réguler un appareil médical de traitement du sang (4) afin d'exécuter un procédé d'élimination du sang et/ou d'un mélange sanguin d'un circuit sanguin extracorporel (1) utilisé pour le traitement du sang d'un patient avec un filtre à sang (19) après l'achèvement d'une séance de traitement du sang lors d'un processus de réinjection, où le filtre à sang (19) comprend une chambre à sang (19a) ainsi qu'une chambre à dialysat (19b), entre lesquelles est agencée une membrane, où, pour le traitement du sang, la chambre à sang (19a) est reliée à un conduit sanguin artériel (9) menant à la chambre à sang (19a) et à un conduit sanguin veineux (23) partant de la chambre à sang (19a), ainsi qu'à un conduit d'alimentation en liquide de dialyse (31a) menant à la chambre à dialysat (19b) et à un conduit d'évacuation de dialysat (31b) partant de la chambre à dialysat (19b), le procédé comprenant l'étape suivante :

   - déplacement du sang et/ou du mélange sanguin hors de la chambre à sang (19a) en introduisant un substitut dans le conduit sanguin artériel (9) sous formation d'un mélange sang-substitut; et
   **caractérisé par** l'étape de

- génération d'une différence de pression dans le filtre à sang (19) avec une pression inférieure dans la chambre à dialysat (19b) et une pression supérieure dans la chambre à sang (19a) lors du processus de réinjection afin d'éliminer l'eau du mélange sang-substitut via le filtre à sang (19).

2. Le dispositif de commande et/ou de régulation (29) selon la première revendication, où la différence de pression est générée, au moins en partie, par au moins une pompe, notamment une pompe à ultrafiltration (40), une pompe à substitut (17) et/ou une pompe à sang (11).

3. Le dispositif de commande et/ou de régulation (29) selon la revendication 2, où la pompe à ultrafiltration (40) est activée, au moins temporairement, simultanément avec la pompe à substitut (17) et/ou la pompe à sang (11).

4. Le dispositif de commande et/ou de régulation (29) selon l'une quelconque des revendications précédentes, où le débit ($Q_{BP}$) de la pompe à sang (11) et/ou le débit ($Q_{Substituatpumpe}$) de la pompe à substitut (17) est compris entre 30 and 280 ml/min.

5. Le dispositif de commande et/ou de régulation (29) selon l'une quelconque des revendications précédentes, où le rapport des débits ($Q_{UF}/Q_{BP}$) de la pompe à ultrafiltration (40) à la pompe à sang (11) et/ou le rapport des débits ($Q_{UF}/Q_{Substituatpumpe}$) de la pompe à ultrafiltration (40) à la pompe à substitut (17) se situe(ent) dans une plage de valeurs de 0,01 à 0,8.

6. Le dispositif de commande et/ou de régulation (29) selon l'une quelconque des revendications précédentes, où le rapport ($Q_{UF}/Q_{BP}$) et/ou le rapport ($Q_{UF}/Q_{Substituatpumpe}$) augmente(ent) pendant l'exécution du procédé en fonction du volume de substitut acheminé jusqu'ici ou depuis le début du procédé.

7. Le dispositif de commande et/ou de régulation (29) selon l'une quelconque des revendications précédentes, où le rapport ($Q_{UF}/Q_{BP}$) et/ou le rapport ($Q_{UF}/Q_{Substituatpumpe}$) est/sont modifié(s) pendant l'exécution du procédé afin de commander ou de réguler l'hématocrite (HKT) dans le conduit sanguin veineux (23) à une valeur prédéterminée.

8. Le dispositif de commande et/ou de régulation (29) selon l'une quelconque des revendications précédentes, où le rapport ($Q_{UF}/Q_{BP}$) et/ou le rapport ($Q_{UF}/Q_{Substituatpumpe}$) est/sont régulé (s) en fonction de l'hématocrite (HTK) se trouvant dans le conduit sanguin veineux (23), qui est déterminée au moyen d'un dispositif de détection, notamment au moyen d'un détecteur (25) de substitut sanguin veineux, et en fonction d'un volume de substitut prédéterminé à économiser.

9. Un appareil médical de traitement du sang (4) ayant

   - éventuellement au moins un circuit sanguin extracorporel (1) avec un intérieur de conduit;
   - au moins une pompe à sang (11) agencée sur ou dans le circuit sanguin extracorporel (1) pour acheminer le sang dans l'intérieur de conduit du circuit sanguin extracorporel (1);
   **caractérisé par**
   - au moins un dispositif de commande et/ou de régulation (29) selon l'une quelconque des revendications 1 à 8.

10. L'appareil médical de traitement du sang (4) selon la revendication 9, qui comprend ou est relié à, de préférence en communication fluidique, au moins une pompe pour générer une différence de pression dans le filtre à sang (19), avec une pression inférieure dans la chambre à dialysat (19b) et une pression supérieure dans la chambre à sang (19a).

11. Un support de stockage numérique, notamment sous la forme d'une disquette, d'un CD ou d'un DVD ou d'un EPROM, avec des signaux de commande lisibles électroniquement, configuré pour interagir avec un système informatique programmable d'un appareil médical de traitement du sang de sorte que les étapes mécaniques mentionnées dans l'une quelconque des revendications 1 à 8 soient déclenchées.

12. Un produit de programme d'ordinateur ayant un code de programme enregistré sur un support lisible par une machine pour déclencher les étapes mécaniques mentionnées dans l'une quelconque des revendications 1 à 8 lorsque le produit de programme d'ordinateur s'exécute sur un ordinateur d'un appareil médical de traitement du sang.

13. Un programme d'ordinateur ayant un code de programme pour déclencher les étapes mécaniques mentionnées dans l'une quelconque des revendications 1 à 8 lorsque le programme d'ordinateur s'exécute sur un ordinateur d'un appareil médical de traitement du sang.

Fig. 1

Fig. 2

Fig. 3

EP 3 515 530 B1

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008028579 A1
- DE 102011108785 A1
- WO 2010121750 A1
- EP 2583701 A1